(19) Europäisches Patentamt
European Patent Office
Office européen des brevets

(11) EP 0 989 136 A1

(12) **EUROPEAN PATENT APPLICATION**
published in accordance with Art. 158(3) EPC

(43) Date of publication:
**29.03.2000 Bulletin 2000/13**

(21) Application number: **98919612.6**

(22) Date of filing: **15.05.1998**

(51) Int. Cl.7: **C07K 14/82**
// C12N15/09, A61K38/17

(86) International application number:
**PCT/JP98/02148**

(87) International publication number:
**WO 98/51707 (19.11.1998 Gazette 1998/46)**

(84) Designated Contracting States:
**AT BE CH CY DE DK ES FI FR GB GR IE IT LI LU MC NL PT SE**

(30) Priority: **15.05.1997 JP 12611397**

(71) Applicant:
**KYOWA HAKKO KOGYO CO., LTD.**
**Chiyoda-ku, Tokyo 100-8185 (JP)**

(72) Inventors:
- **SHIBATA, Kenji**
  **Tokyo 206-0041 (JP)**
- **YAMASAKI, Motoo**
  **Tokyo 194-0021 (JP)**
- **YOSHIDA, Tetsuo**
  **Tokyo 194-0022 (JP)**
- **MIZUKAMI, Tamio**
  **Tokyo 194-0032 (JP)**
- **SHINKAI, Akeo**
  **Tokyo 194-0021 (JP)**
- **ANAZAWA, Hideharu**
  **Tokyo 178-0064 (JP)**

(74) Representative:
**VOSSIUS & PARTNER**
**Siebertstrasse 4**
**81675 München (DE)**

(54) **PEPTIDES HAVING CYCLIC STRUCTURES AND EXERTING P53 PROTEIN ACTIVTY-RESTORING EFFECT ON P53 PROTEIN MUTANTS**

(57) Peptides having cyclic structures represented by the following general formula(I): $R^1(X^1)^{n1}(X^2)^{n2}(X^3)^{n3}(X^4)^{n4}(X^5)^{n5}(X^6)^{n6}(X^7)^{n7}(X^8)^{n8}(X^9)^{n9}(X^{10})^{n10}(x^{11})n^{11}(X^{12})^{n12}(X^{13})^{n13}(X^{14})^{n14}(X^{15})^{n15}(X^{16})^{n16}(X^{17})^{n17}R2$ and exerting the effect of restoring a P53 protein activity on P53 protein mutants or pharmacologically acceptable salts thereof. In said formula (I) $X^i$ means an arbitrary member selected from among $X^1$ to $X^{17}$ while $n^i$ means an arbitrary member selected from among $n^1$ to $n^{17}$ (provided that i is an integer of from 1 to 17), then $X^i$ represents an amino acid residue or an organic acid residue; a functional group in a residue $X^p$ selected among $X^1$ to $X^{11}$ (provided that p is an integer of from 1 to 11) and a functional group $X^q$ selected from among $X^8$ to $X^{17}$ (provided that q is an integer of from 8 to 17 and larger than p) form a cyclic structure having a crosslinkage selected from among S-S, S-$CH_2$-S, S-$CH_2$-$C_6H_4$,-$CH_2$-S, S-$CH_2$-CO, CO-NH, NH-CO, O-CO or CO-O; $R^1$ represents optionally substituted alkanoyl, etc.; and $R^2$ represents optionally substitued alkoxy, etc.

EP 0 989 136 A1

## Description

Technical Field

The present invention relates to peptides having a cyclic structure and having the activity to restore the activities of P53 protein to mutant P53 protein, and pharmaceutically acceptable salts thereof. The peptides comprise, as a main structure, a sequence in the C-terminal domain of human P53 protein, or a sequence obtained by introducing a mutation into a part thereof. The peptides bind to mutant P53 protein which lost specific DNA-binding activity by point mutation, and restore the specific DNA-binding activity thereto. As the peptides are capable of restoring the P53 protein-dependent transcription activity, they are expected to be useful as therapeutic agents for cancer.

Technical Background

The p53 gene is the most commonly mutated tumor suppressor gene identified in human cancers. The gene has been found to be mutated in most kinds of cancers, e.g. breast cancer, colon cancer and lung cancer, and it is considered that the gene is mutated in more than 50% of all cancer patients [Science, 253, 49 (1991)]. It is also known that, in many kinds of tumors, there is a correlation between the existence of p53 mutation and the malignancy, prognosis and occurrence of metastasis, and p53-deficient mice are susceptible to tumors; thus, it is evident that p53 has an important function in tumor suppression [Nature, 356, 215 (1992)].

The main biological functions of the p53 gene are (1) to arrest the cell cycle of a cell having DNA damage at the G1 phase, (2) to lead such a cell to apoptosis (cell death), and the like. These functions are lost in mutant p53 found in cancer cells. In recent years, it has become apparent that normal P53 protein (hereinafter P53 refers to the protein which is the product of expression of the p53 gene) functions as a transcription factor which binds to specific nucleotide sequences and activates transcription [Cell, 78, 543 (1994)]. It is known that P53 activates transcription of genes such as p21waf1 (CDK inhibitor), GADD45 and bax, which are considered to play an important role in leading the cell to cell cycle arrest at the G1 phase or to apoptosis [Cell, 75, 817 (1993); ibid., 71, 587-597 (1992); ibid., 80, 293 (1995)].

In order to suppress cancer by treating cancer cells carrying mutant p53/P53 which have lost the above DNA-binding activity and transcription-activating activity and thereby restoring such activities, some attempts of gene therapy have been made, in which wild-type p53 gene was introduced into cells. For example, it has been reported that introduction of wild-type p53 gene alone or in combination with known antitumor agents is effective on human lung cancer and head and neck cancer implanted into mice [Cancer Research, 54, 2287 (1994); ibid., 55, 1 (1995)]. It has also been reported that direct introduction of wild-type p53 gene is effective on human non-small cell lung cancer, and clinical effectiveness on cancers of restoration of the activities of p53/P53 in cells has been proved [Nature Med., 2, 985 (1996)]. However, gene therapy is subject to considerable restrictions and thus a need exists for an excellent and less complicated method for restoring the p53/P53 activities to cells carrying mutant p53/P53.

Unlike the mutations in other tumor suppressor genes, the mutations in p53 gene are mostly missense (point) mutation, and deletion or insertion mutation is not common. It is considered that substitution of one amino acid residue causes a change in the higher-order structure of P53 to lead to loss of the above activities of binding to specific DNA sequences and activating transcription, whereby cell cycle arrest at the G1 phase or apoptosis of a cell having DNA damage is not induced [Science, 256, 827 (1992)]. About 90% of the missense mutations of p53 in human cancers are located in exons 5-8 (codons 126-306) among 11 exons; particularly, the frequency of mutation is very high at sites called hot spots such as codons 143, 175, 245, 248, 249, 273 and 282, though the frequency slightly varies depending on the part at which cancer is produced [Biochemica Biophysica Acta, 1155, 181 (1993); Cancer Research, 54, 4855 (1994)]. From the result of X-ray crystal structure analysis of the DNA-binding region of P53, amino acid substitutions in the hot spots are supposed to take place at (1) the residues directly acting on the bases in DNA chain or the backbones (e.g. codons 248 and 273) and (2) the residues contributing to stabilization of the structure of the DNA-binding region (e.g. codons 143, 175, 249 and 282) [Science, 265, 346 (1994)]. Whichever residues of (1) and (2) are substituted, these point mutations in the DNA-binding region of P53 result in loss of its activity of binding to specific DNA sequences. Accordingly, a substance interacting with P53 which has lost the specific DNA-binding activity by these point mutations or other mutations and capable of restoring its DNA-binding activity is expected to possess antitumor activity.

Monoclonal antibody Pab421, whose epitope is a sequence in the C-terminal domain of P53, and DnaK, which is a heat shock protein of Escherichia coli, have been reported as substances capable of restoring the DNA-binding activity to mutant P53 [Nucleic Acids Research, 21, 3167 (1993)]. Recently, it has been demonstrated that the DNA-binding activity can be restored to some mutants of p53 by further mutating a residue interacting with DNA of mutant P53 (substitution of 284Thr with Arg) [Nature Medicine, 2, 1143 (1996)]. It has also been reported that the introduction of Pab421 or Arg284 mutation induces restoration of the transcription-activating ability to some mutants of p53 protein [Cancer Research, 55, 3490 (1995); Nature Medicine, 2, 1143 (1996)]. These results suggest that direct treatment of

mutant P53 is capable of restoring the DNA-binding, thereby restoring the transcription-activating activity, and as a result, may enable the mutant to have antitumor activity. As to the low molecular compounds having such ability, geldanamycin is reported to alter the conformation of mutant p53 in cells and partially restores its ability to bind to specific DNA, but the compound is considered to act indirectly, probably via HSP90 protein [Oncogene, 11, 933 (1995); Proceedings of the National Academy of Science USA, 93, 8379 (1996)]. Further, ellipticine is reported to have inhibitory activity on an enzyme contributing to phosphorylation of P53 and inhibit phosphorylation of mutant P53 expressed in large amounts in cancer cells, thereby selectively inducing apoptosis of cancer cells (Japanese Published Unexamined Patent Application No. 279441/94).

As to the regulation mechanism for transcriptional activation of P53, it is considered that the highly basic C-terminal sequence has the negative allosteric regulation effect on the DNA-binding activity, because the DNA-binding activity of wild-type P53 is enhanced by deletion of the C-terminal 30 amino acids [Nucleic Acids Research, 21, 3167 (1993); Nature Medicine, 2, 1143 (1996)]. Also from the fact that this C-terminal sequence is the epitope for the above-mentioned monoclonal antibody Pab421 restoring the DNA-binding activity to mutant P53, it is supposed that the C-terminal sequence supresses the DNA-binding activity of P53 by interaction with the DNA-binding region of P53 intra- or intermolecularly. On peptides derived from the C-terminal domain of P53 containing this epitope, the report has been made that such peptides consisting of 12-15 amino acid residues enhance the DNA-binding ability of wild-type P53 expressed in Escherichia coli or yeast cells [(Cell, 83, 237 (1995); Oncogene, 12, 921 (1996)]. It has recently been reported that a peptide consisting of 13 amino acid residues in the C-terminal domain of P53 (codons 371-383) is capable of partially restoring the in vitro specific DNA-binding activity to human P53 wherein codon 273 has been mutated to His (hereinafter referred to as P53His273) (WO 96/25434). However, in this report, the peptide is used at a very high concentration of 0.4 mM to give such result, and neither the capability of this peptide to restore the activity to mutant P53 in cells nor its effect on mutant P53 other than His273 is demonstrated. It is also not clear whether this peptide is capable of restoring the transcription activity and growth inhibitory activity to mutant P53 in cells, either. It has also been reported that microinjection of a peptide comprising the C-terminal sequence of P53 (codons 369-382) wherein lysine at one position (corresponding to Lys381) has been polyethylene glycol (PEG)-modified into cell strain SW480 derived from human colon cancer (having the mutations of 273Arg to His and 309Pro to Ser) results in restoration of the transcription activity of P53 derived from said cell [Oncogene, 13, 2477 (1996)].

## Disclosure of the Invention

The present invention provides a peptide having a cyclic structure and having the activity to restore the DNA-binding activity or the P53 protein-dependent transcription activity to mutant P53 protein, and a pharmaceutically acceptable salt thereof, said peptide being represented by general formula (I):

$$R^1(X^1)^{n1}(X^2)^{n2}(X^3)^{n3}(X^4)^{n4}(X^5)^{n5}(X^6)^{n6}(X^7)^{n7}(X^8)^{n8}(X^9)^{n9}(X^{10})^{n10}(X^{11})^{n11}(X^{12})^{n12}(X^{13})^{n13}(X^{14})^{n14}(X^{15})^{n15}(X^{16})^{n16}(X^{17})^{n17}R^2 \quad (I)$$

{wherein any of $X^1$ to $X^{17}$ and n1 to n17 may be denoted by $X^i$ and ni, respectively (i stands for an integer of 1 to 17); $X^i$ represents an amino acid residue or an organic acid residue as defined below; ni represents 0 or 1; $(X^i)^{ni}$ represents $X^i$ when ni is 1, and represents a bond when ni is 0; 7 to 17 different $X^i$s (ni=1) are selected, arranged in order of increasing number i, and bonded to one another, with $R^1$ bonded to the N-terminus and $R^2$ bonded to the C-terminus, to represent one sequence, in which a functional group in residue $X^p$ (p is an integer of 1 to 11) is selected from the group of $X^1$ to $X^{11}$ and a functional group in residue $X^q$ (q is an integer of 8 to 17, provided that q is larger than p) is selected from the group of $X^8$ to $X^{17}$ form a cyclic structure; $R^1$ represents substituted or unsubstituted alkanoyl, substituted or unsubstituted alkoxycarbonyl, substituted or unsubstituted aralkyloxycarbonyl, substituted or unsubstituted aryloxycarbonyl, substituted or unsubstituted aroyl, 9-fluorenylmethoxycarbonyl, or hydrogen; $X^1$ represents a residue of 2-mercaptobenzoic acid, 3-mercaptopropionic acid, 4-mercaptobutanoic acid, mercaptoacetic acid, adipic acid, suberic acid, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid , p-aminophenylalanine, serine, threonine, homoserine, α-methylserine, 3-hydroxyproline or 4-hydroxyproline; $X^2$ represents a residue of leucine, isoleucine, valine, alanine, norvaline, norleucine, 2-aminobutanoic acid, homoleucine, β-alanine, α-aminoisobutanoic acid, β-cyclopropylalanine, β-chloroalanine, 1-aminocyclopentane-1-carboxylic acid, 1-amino-1-cyclohexanecarboxylic acid, 2-amino-1-cyclopentanecarboxylic acid, t-butylglycine, diethylglycine, t-butylalanine, O-methylserine, cyclohexylglycine, cyclohexylalanine or glycine; $X^3$ represents lysine, arginine, ornithine, 2,4-diaminobutanoic acid, 2,3-diaminopropionic acid, p-aminophenylalanine or glycine; $X^4$ represents a residue of serine, threonine, homoserine, α-methylserine, 3-hydroxyproline, 4-hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine,

glycine, 2-mercaptobenzoic acid, 3-mercaptopropionic acid, 4-mercaptobutanoic acid, mercaptoacetic acid, adipic acid or suberic acid; $X^5$ represents lysine, arginine, ornithine, 2,4-diaminobutanoic acid, 2,3-diaminopropionic acid, p-aminophenylalanine or glycine; $X^6$ represents a residue of lysine, arginine, ornithine, 2,4-diaminobutanoic acid, 2,3-diaminopropionic acid, p-aminophenylalanine or glycine; $X^7$ represents a residue of alanine, β-alanine, 2-aminobenzoic acid, 3-aminobenzoic acid, 4-aminobenzoic acid, 3-aminomethylbenzoic acid, proline, 3-hydroxyproline, 4-hydroxyproline, L-1,2,3, 4-tetrahydroisoquinoline-7-carboxylic acid, cysteine, homocysteine, penicillamine, 2,3-diaminopropionic acid, 2,4-diaminobutanoic acid, ornithine, lysine, p-aminophenylalanine, aspartic acid, glutamic acid, isoaspartic acid, isoglutamic acid, 2-aminoadipic acid, 2-aminosuberic acid or glycine; $X^8$ represents glutamine, asparagine, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, serine, threonine, homoserine, α-methylserine, 3-hydroxyproline, 4-hydroxyproline, glycine, 2-mercaptobenzoic acid, 3-mercaptopropionic acid, 4-mercaptobutanoic acid, mercaptoacetic acid, adipic acid or suberic acid; $X^9$ represents a residue of serine, threonine, homoserine, α-methylserine, 3-hydroxyproline, 4-hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, glycine, 2-mercaptobenzoic acid, 3-mercaptopropionic acid, 4-mercaptobutanoic acid, mercaptoacetic acid, adipic acid or suberic acid; $X^{10}$ represents a residue of serine, threonine, homoserine, α-methylserine, hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, glycine, 2-mercaptobenzoic acid, 3-mercaptopropionic acid, 4-mercaptobutanoic acid, mercaptoacetic acid, adipic acid or suberic acid; $X^{11}$ represents a residue of serine, threonine, homoserine, α-methylserine, hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, glycine, 2-mercaptobenzoic acid, 3-mercaptopropionic acid, 4-mercaptobutanoic acid, mercaptoacetic acid, adipic acid or suberic acid; $X^{12}$ represents lysine, arginine, ornithine, 2,4-diaminobutanoic acid, 2,3-diaminopropionic acid, p-aminophenylalanine or glycine; $X^{13}$ represents a residue of histidine, alanine, 4-thiazolylalanine, 2-thienylalanine, 2-pyridylalanine, 3-pyridylalanine, 4-pyridylalanine, (3-N-methyl)piperidylalanine, 3-(2-quinoyl)alanine, serine, threonine, homoserine, α-methylserine, 3-hydroxyproline, 4-hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine or glycine; $X^{14}$ represents a residue of lysine, arginine, ornithine, 2,4-diaminobutanoic acid, 2,3-diaminopropionic acid, p-aminophenylalanine, serine, threonine, homoserine, α-methylserine, 3-hydroxyproline, 4-hydroxyproline, cysteine, homocysteine, penicillamine, aspartic acid, glutamic acid, homoglutamic acid, isoaspartic acid, isoglutamic acid, 2-aminoadipic acid, 2-aminosuberic acid or glycine, and an amino group or guanidino group in the side chain of $X^{14}$ may be modified with $R^3$ ($R^3$ has the same significance as $R^1$); $X^{15}$ represents a residue of lysine, arginine, ornithine, 2,4-diaminobutanoic acid, 2,3-diaminopropionic acid, p-aminophenylalanine or glycine; $X^{16}$ represents a residue of leucine, alanine, 4-thiazolylalanine, 2-thienylalanine, isoleucine, norleucine, homoleucine, valine, norvaline, β-alanine, α-aminoisobutanoic acid, 2-aminobutanoic acid, β-cyclopropylalanine, β-chloroalanine, 1-aminocyclopentane-1-carboxylic acid, 1-amino-1-cyclohexanecarboxylic acid, 2-amino-1-cyclopentanecarboxylic acid, t-butylglycine, diethylglycine, t-butylalanine, O-methylserine, cyclohexylglycine, cyclohexylalanine or glycine; $X^{17}$ represents a residue of 2-mercaptoaniline, cysteamine, homocysteamine, cysteine, homocysteine, penicillamine, ornithine, lysine, 2,3-diaminopropionic acid, 2,4-diaminobutanoic acid, p-aminophenylalanine, glutamic acid, aspartic acid, homoglutamic acid, isoaspartic acid, isoglutamic acid, 2-aminoadipic acid or 2-aminosuberic acid; $R^2$ represents substituted or unsubstituted alkoxy, substituted or unsubstituted aralkyloxy, amino, substituted or unsubstituted alkylamino, substituted or unsubstituted dialkylamino, substituted or unsubstituted aralkylamino, substituted or unsubstituted arylamino, or hydroxy; and one to several residues which are the same or different and arbitrarily selected from the group consisting of organic acid residues, amino acid residues and a 12-aminododecanoic acid residue mentioned in the above $X^i$ representations may be deleted, substituted or added at arbitrary positions in the sequence}.

The compounds represented by formula (I) are hereinafter referred to as Compounds (I).

In the definitions of $R^1$ and $R^2$ in formula (I), the alkyl moiety of the alkanoyl, the alkoxy, the alkoxycarbonyl, the alkylamino and the dialkylamino includes straight-chain or branched alkyl having 1 to 20 carbon atoms and alicyclic alkyl having 3 to 8 carbon atoms. Examples of the straight-chain or branched alkyl having 1 to 20 carbon atoms are methyl, ethyl, propyl, isopropyl, butyl, isobutyl, sec-butyl, tert-butyl, pentyl, isopentyl, neopentyl, hexyl, heptyl, octyl, nonyl, decyl, undecyl, dodecyl, hexadecyl and octadecyl. Examples of the alicyclic alkyl having 3 to 8 carbon atoms are cyclopropyl, cyclobutyl, cyclopentyl, cyclohexyl, cycloheptyl and cyclooctyl. The aryl moiety of the aryloxycarbonyl, the aroyl and the arylamino includes phenyl and naphthyl. The aralkyl moiety of the aralkyloxycarbonyl, the aralkyloxy and the aralkylamino includes benzyl, phenethyl, benzhydryl and trityl having 7 to 20 carbon atoms. The substituted alkanoyl, the substituted alkoxy, the substituted alkoxycarbonyl, the substituted alkylamino, the substituted

dialkylamino, the substituted aryloxycarbonyl, the substituted aroyl, the substituted arylamino, the substituted aralkyloxycarbonyl, the substituted aralkyloxy and the substituted aralkylamino each have 1 to 3 substituents which are the same or different. Examples of the substituents are halogen, nitro, alkyl, aryl, hydroxy, alkoxy, amino, alkylamino, dialkylamino, aryloxy, carboxy, alkoxycarbonyl, aryloxycarbonyl and a heterocyclic group. The halogen means fluorine, chlorine, bromine or iodine. The alkyl moiety of the alkyl, the alkoxy, the alkylamino, the dialkylamino and the alkoxycarbonyl and the aryl moiety of the aryl, the aryloxy and the aryloxycarbonyl have the same meanings as defined above. The heterocyclic group includes 3 to 8-membered alicyclic or aromatic heterocyclic groups having at least one hetero atom such as oxygen, sulfur or nitrogen, polycyclic groups formed by condensation of such a heterocyclic group, and polycyclic groups formed by condensation of such a heterocyclic group and aryl. Examples of the heterocyclic groups are imidazolyl, pyridyl, indolyl, quinolyl, isoquinolyl, pyrrolidinyl, piperidinyl, piperazinyl, morpholino, thiomorpholino and homopiperazinyl.

The expression "one to several residues may be deleted, substituted or added in the sequence" herein means that the sequence may contain deletion, substitution or addition of a single or plural amino acid residues or organic acid residues mentioned above or substitution or addition of a 12-aminododecanoic acid residue at a single or plural arbitrarily selected positions therein, and the total number of such residues deleted, substituted or added is one to several, which deletion, substitution and addition may be simultaneously contained in the sequence. The expression "one to several residues may be added in the sequence" means that only addition may be contained in the sequence in the same manner as above. The number of residues deleted, substituted or added is preferably 1 to 10, more preferably 1 to 4.

The pharmaceutically acceptable salts of Compounds (I) include acid addition salts, metal salts, organic base addition salts, etc. Examples of the pharmaceutically acceptable acid addition salts are inorganic acid addition salts such as hydrochloride, sulfate and phosphate, and organic acid addition salts such as acetate, maleate, fumarate, tartrate and citrate. Examples of the pharmaceutically acceptable metal salts are alkali metal salts such as sodium salt and potassium salt, alkaline earth metal salts such as magnesium salt and calcium salt, aluminum salt and zinc salt. Examples of the pharmaceutically acceptable organic base addition salts are salts with primary amines, e.g. methylamine, ethylamine or aniline, secondary amines, e.g. dimethylamine, diethylamine, pyrrolidine, piperidine, morpholine or piperazine, and tertiary amines, e.g. trimethylamine, triethylamine, N,N-dimethylaniline or pyridine, and ammonium salt.

The following abbreviations for amino acids and their protecting groups used herein follow the recommendations by IUPAC-IUB Joint Commission on Biochemical Nomenclature [European Journal of Biochemistry, 138, 9 (1984)].

The following abbreviations represent the corresponding following amino acids and organic acids, unless otherwise specified.

Gly: Glycine
Thr: L-Threonine
Gln: L-Glutamine
Glx: L-Glutamic acid or L-glutamine
Leu: L-Leucine
Ser: L-Serine
Lys: L-Lysine
Arg: L-Arginine
Asp: L-Aspartic acid
His: L-Histidine
Cys: L-Cysteine
Add: 12-Aminododecanoic acid

Preferred examples of the peptides having a cyclic structure according to the present invention are shown below. Preferred examples of the peptides having a cyclic structure according to the present invention:

(1) A peptide wherein the cyclic structure is formed by a S-S, S-$CH_2$-S, S-$CH_2$-$C_6H_4$-$CH_2$-S, S-$CH_2$-CO, CO-NH, NH-CO, O-CO or CO-O bond between $X^p$ and $X^q$
(2) A peptide of (1) wherein $X^p$ (np=1) is an N-terminal residue and $X^q$ (nq=1) is a C-terminal residue
(3) A peptide of (1) wherein $X^p$ (np=1) is not an N-terminal residue and $X^q$ (nq=1) is not a C-terminal residue
(4) A peptide of (1) wherein $X^p$ (np=1) is not an N-terminal residue and $X^q$ (nq=1) is a C-terminal residue
(5) A peptide of (1) wherein $X^p$ (np=1) is an N-terminal residue and $X^q$ (nq=1) is not a C-terminal residue
(6) A peptide of (2) wherein $X^p$ (np=1) is $X^1$ and $X^q$ (nq=1) is $X^{17}$
(7) A peptide of (5) wherein $X^p$ (np=1) is $X^1$ and $X^q$ (nq=1) is $X^{17}$
(8) A peptide of (5) wherein $X^p$ (np=1) is $X^1$ and $X^q$ (nq=1) is $X^{16}$

(9) A peptide of (5) wherein $X^p$ (np=1) is an N-terminal residue and $X^q$ (nq=1) is $X^8$
(10) A peptide of (3) wherein $X^p$ (np=1) is $X^8$ and $X^q$ (nq=1) is $X^{14}$
(11) A peptide of (4) wherein $X^p$ (np=1) is $X^3$ and $X^q$ (nq=1) is a C-terminal residue
(12) A peptide of (3) wherein $X^p$ (np=1) is $X^3$ and $X^q$ (nq=1) is not a C-terminal residue
(13) A peptide of (5) wherein $X^p$ (np=1) is an N-terminal residue and $X^q$ (nq=1) is $X^{11}$

Particularly preferred are peptides of (6)-(13) above.
Preferred examples of Compounds (I) are shown in Tables 1-1 and 1-2 together with straight-chain peptides analogous thereto as reference examples. Particularly preferred are Compounds 4-8, 11 and 17-24.

**Table 1-1**

| Compound No. | Structure |
|---|---|
| 1 | H-LKSKKGQSTSRHKKL-OH |
| 2 | H-KSKKGQSTSRHKK-OH |
| 3 | H-KKGQSTSRHKK-OH |
| 4 | H-CLKSKKGQSTSRHKKLC-OH |
| 5 | LKSKKGQSTSRHKKL |
| 6 | H-CLKSKKGQSTSRHKKLC-$NH_2$ |
| 7 | Ac-CLKSKKGQSTSRHKKLC-$NH_2$ |
| 8 | H-CLKSKKGQSTSRHKKLC-NH-$(CH_2)_{11}$ -$CONH_2$ |
| 9 | H-CLKSKKGQSTSRHKKLCNH-$(CH_2)_{11}$ -$CH_3$ |
| 10 | H-CLKSKKGQSTSRHKKLC-NH-$(CH_2)_{17}$-$CH_3$ |
| 11 | H-CLKSKKGQSTSRHKKLC-$NH_2$<br>Ac |
| 12 | $(CH_2)$<br>H-CLKSKKGQSTSRHKKLC-$NH_2$ |
| 13 | H-KLKSKKGQSTSRHKKL |
| 14 | H-CLKSKKGCSTSRHKKL-$NH_2$ |
| 15 | H-KLKSKKGDSTSRHKKL-$NH_2$ |
| 16 | H-LKSKKGDSTSRHKKL-$NH_2$ |

The amino acid residues are represented by the following letters: G, Gly; T, Thr; D, Asp; Q, Gln; L, Leu; S, Ser; K, Lys; R, Arg; H, His; C, Cys.

**Table 1-2**

| Compound No. | Structure |
|---|---|
| 17 | H-CLKSKKQSTSRHKKLC-$NH_2$ |
| 18 | $CH_2CO$-LKSKKGQSTSRHKKLC-$NH_2$ |
| 19 | ($CH_2$-(o-$C_6H_4$)-$CH_2$) H-CLKSKKGQSTSRHKKLC-$NH_2$ |
| 20 | H-LKCKKGQSTSRHKKLC-$NH_2$ |
| 21 | H-CLKSKKGQSTCRHKKL-$NH_2$ |
| 22 | H-LKCKKGQSTCRHKKL-$NH_2$ |
| 23 | H-CLKSKKGQSTSRHKKLCG-NH-$(CH_2)_3$-$CH_3$ |
| 24 | H-LKDKKGQSTSRHKKL-$NH_2$ |

The amino acid residues are represented by the following letters: G, Gly; D, Asp; Q, Gln; L, Leu; T, Thr; S, Ser; K, Lys; R, Arg; C, Cys; H, His.

Compound Nos. 1 to 24 respectively correspond to SEQ ID NOS as follows: Compound 1, SEQ ID NO: 1; Compound 2, SEQ ID NO: 2; Compound 3, SEQ ID NO: 3; Compound 4, SEQ ID NO: 4; Compound 5, SEQ ID NO: 5; Compound 6, SEQ ID NO: 6; Compound 7, SEQ ID NO: 7; Compound 8, SEQ ID NO: 16; Compound 9, SEQ ID NO: 17; Compound 10, SEQ ID NO: 18; Compound 11, SEQ ID NO: 19; Compound 12, SEQ ID NO: 20; Compound 13, SEQ ID NO: 21; Compound 14, SEQ ID NO: 22; Compound 15, SEQ ID NO: 23; Compound 16, SEQ ID NO: 24; Compound 17, SEQ ID NO: 25; Compound 18, SEQ ID NO: 26; Compound 19, SEQ ID NO: 27; Compound 20, SEQ ID NO: 28; Compound 21, SEQ ID NO: 29; Compound 22, SEQ ID NO: 30; Compound 23, SEQ ID NO: 31; compound 24, SEQ ID NO: 32.

The following abbreviations represent the corresponding following amino-acid-protecting groups and side-chain-protected amino acids.

Fmoc: 9-Fluorenylmethyloxycarbonyl
t-Bu: t-Butyl
Trt: Trityl
Pmc: 2,2,5,7,8-Pentamethylchroman-6-sulfonyl
Boc: t-Butyloxycarbonyl
Mtt: 4-Methyltrityl
Aloc: Allyloxycarbonyl
Al: Allyl
Fmoc-Thr(t-Bu)-OH: $N^{\alpha}$-9-Fluorenylmethyboxycarbonyl-O-t-butyl-L-threonine
Fmoc-Thr(Trt)-OH: $N^{\alpha}$-9-Fluorenylmethyloxycarbonyl-O-trityl-L-threonine
Fmoc-Ser(t-Bu)-OH: $N^{\alpha}$-9-Fluorenylmethyloxycarbonyl-O-t-butyl-L-serine
Fmoc-Ser(Trt)-OH: $N^{\alpha}$-9-Fluorenylmethyloxycarbonyl-O-trityl-L-serine
Fmoc-Lys(Boc)-OH: $N^{\alpha}$-9-Fluorenylmethyloxycarbonyl-$N^{\varepsilon}$-t-butyloxycarbonyl-L-lysine
Fmoc-Gln(Trt)-OH: $N^{\alpha}$-9-Fluorenylmethyloxycarbonyl-$N^{\delta}$-trityl-L-glutamine

Fmoc-Arg (Pmc)-OH: $N^{\alpha}$-9-Fluorenylmethyloxycarbonyl-$N^{g}$-2,2,5,7,8-pentamethylchroman-6-sulfonyl-L-arginine
Fmoc-His(Trt)-OH: $N^{\alpha}$-9-Fluorenylmethyloxycarbonyl-$N^{im}$-trityl-L-histidine
Fmoc-Cys (Trt)-OH: $N^{\alpha}$-9-Fluorenylmethyloxycarbonyl-S-trityl-L-cysteine
Fmoc-Lys(Mtt)-OH: $N^{\alpha}$-9-Fluorenylmethyloxycarbonyl-$N^{\varepsilon}$-4-methyltrityl-L-lysine
Boc-Cys(Trt)-OH: $N^{\alpha}$-t-Butyloxycarbonyl-S-trityl-L-cysteine
Fmoc-Lys(Aloc)-OH: $N^{\alpha}$-9-Fluorenylmethyloxycarbonyl-$N^{\varepsilon}$-allyloxycarbonyl-L-lysine
Fmoc-Asp(OAl)-OH: N-9-Fluorenylmethyloxycarbonyl-L-aspartic acid-β-allyl ester

The following abbreviations represent the corresponding following reaction solvents, reaction reagents, etc.

PyBOP: Benzotriazol-1-yloxytripyrrolidinophosphonium hexafluorophosphate
HBTU: 2-(1H-Benzotriazol-1-yl)-1,1,3,3-tetramethyluronium hexafluorophosphate
HATU: O-(7-Azabenzotriazol-1-yl)-1,1,3,3-tetramethyluronium hexafluorophosphate
HOBt: N-Hydroxybenzotriazole
HONSu: N-Hydroxysuccinimide
NMM: N-Methylmorpholine
DMF: N,N-Dimethylformamide
DCM: Dichloromethane
DMSO: Dimethylsulfoxide
NMP: N-Methylpyrrolidone
TFA: Trifluoroacetic acid
DIEA: Diisopropylethylamine
TFE: Trifluoroethanol

The processes for producing Compounds (I) are described below.

Compounds (I) can be synthesized by general liquid phase or solid phase peptide synthetic methods, appropriate combinations thereof, or methods similar thereto [The Peptides, Analysis, Synthesis, Biology, vol. 1, edited by Erhard Gross and Johannes Meinhofer, Academic PreS-S (1979); ibid., vol. 2 (1980); ibid., vol. 3 (1981); Fundamentals and Experiments of Peptide Synthesis, Nobuo Izumiya, et al., Maruzen (1985); Development of Medicines, second series, vol. 14, Peptide Synthesis, compiled under the supervision of Haruaki Yajima, Hirokawa Shoten (1991); International Journal of Peptide and Protein Research, 35, 161 (1990)].

Compounds (I) can also be synthesized by using an automatic peptide synthesizer. That is, the peptide synthesis can be carried out by the use of commercially available peptide synthesizers from Shimadzu Corporation, Applied Biosystems, Inc., USA (hereinafter referred to as ABI), Advanced ChemTech Inc., USA (hereinafter referred to as ACT), etc. using an appropriately side-chain-protected $N^{\alpha}$-Fmoc-amino acid, $N^{\alpha}$-Boc-amino acid, or the like according to respective synthesis programs.

The protected amino acids which are starting materials for the synthesis of Compounds (I) and carrier resins are available from ABI, Shimadzu Corporation, Kokusan Chemical Works Co., Ltd., Nova Biochem Co., Watanabe Chemical Co., Ltd., ACT, Peptide Institute Co., Ltd., etc. The protected amino acids, protected organic acids and protected organic amines which are starting materials for the synthesis of Compounds (I) can be synthesized according to known synthetic methods or methods similar thereto [The Peptides, Analysis, Synthesis, Biology, vol. 1, edited by Erhard Gross and Johannes Meinhofer, Academic Press (1979); ibid., vol. 2 (1980); ibid., vol. 3 (1981); Fundamentals and Experiments of Peptide Synthesis, Nobuo Izumiya, et al., Maruzen (1985); Development of Medicines, second series, vol. 14, Peptide Synthesis, compiled under the supervision of Haruaki Yajima, Hirokawa Shoten(1991); International Journal of Peptide and Protein Research, 35, 161 (1990)].

Cyclization may be carried out after the condensation of all the constituent amino acids and organic acids by a liquid phase or solid phase method, or in the course of condensation process. In the latter case, the obtained cyclization product is subjected to further condensation with amino acids or organic acids to prepare Compound (I).

The cyclic structure may be formed by:

(1) cross-linkage between $x^p$ and $X^q$ general formula (I); or
(2) a CO-NH bond between adjacent amino acids or organic acids which are to be constituents of the cyclic structure.

In carrying out the cyclization reaction, it is preferred to protect functional groups other than those involved in the cyclization with appropriate protecting groups for prevention of possible side reactions.

The general purification steps in organic chemical reactions as used hereinafter refer to neutralization, filtration, extraction, washing, drying, concentration, precipitation, recrystallization, various kinds of chromatography, etc.

(Process 1-1) Cyclization by S-S bond formation

First a peptide having two thiol groups which are protected with appropriate protecting groups is prepared by a solid phase method, a liquid phase method, or a combination thereof. Then, protective groups other than the thiol-protecting groups are removed, followed by removal of the thiol-protecting groups. The thus obtained precursor peptide is subjected to oxidation reaction and the product is purified by general purification steps in organic chemical reactions to give the desired peptide having a cyclic structure with a S-S bond.

1-1-A

The peptide having a cyclic structure with a S-S bond can be prepared by subjecting the above precursor peptide to air oxidation or reaction with an oxidizing agent in an inert solvent. The reaction is carried out in a solvent at a peptide concentration of 0.5-5000 µmol/l, preferably 50-500 µmol/l. As the solvent, buffers such as a 50 mM - 1 M tris (hydroxymethyl ) aminomethane-hydrochloric acid (Tris-HCl) buffer adjusted to pH 4-9, preferably pH 6-8, 5-50% aqueous acetic acid, water, or organic solvents such as DMF, DMSO, acetonitrile, tetrahydrofuran, methanol and ethanol can be used alone or in combination. Examples of the oxidizing agent are potassium ferricyanide and iodine, which are used respectively in the amounts of 0.1-1 time and 0.5-5 times (preferably one time) that of the precursor peptide (weight:weight). DMSO can also be used as the oxidizing agent at a concentration of 10-50%. The reaction is usually completed at 0-40°C for one hour to one week. In some cases, the yield of oxidation product can be increased by addition of glutathione, and the reaction may be carried out in the presence of oxidized glutathione in an amount of 0.5-5 times that of the precursor peptide (weight:weight) and reduced glutathione in an amount of one-half the weight of oxidized glutathione [Journal of American Chemical Society, 103, 5867 (1981); Development of Medicines, second series, vol. 14, Peptide Synthesis, p. 239, compiled under the supervision of Haruaki Yajima, Hirokawa Shoten (1991)]. When iodine is used as the oxidizing agent, zinc powder is added after the completion of reaction until the color of iodine disappears from the reaction mixture, and the mixture is purified as such, or after concentration under reduced pressure, by means of various kinds of chromatography. When potassium ferricyanide is used as the oxidizing agent, the reaction mixture is made weakly acidic by addition of acetic acid and then is purified as such, or after concentration under reduced pressure, by means of various kinds of chromatography. Alternatively, an anion exchange resin such as Dowex 1X2(AcO-) (Dow Chemical Co.) may be added to the reaction mixture to remove excess potassium ferricyanide (ferricyan ion and ferrocyan ion) by adsorption, and then the mixture is purified as such, or after concentration under reduced pressure, by means of various kinds of chromatography.

It is also possible to pyridylsulfenylate or 2-nitropyridylsulfenylate one of the thiol groups and then force the cyclization reaction to proceed to completion simultaneously with the selective removal of the protecting group of the other thiol group [International Journal of Peptide and Protein Research, 29, 162 (1987)]. The solvent for the cyclization reaction, reaction temperature, reaction time, etc. are substantially the same as described above. Further, after the protecting groups of the two thiol groups are removed, an equivalent amount of a reagent for pyridylsulfenylation or 2-nitropyridylsulfenylation may be introduced. Pyridylsulfenylation can also be carried out by adding 1-3 equivalents of a reagent such as 2,2'-dithiodipyridine to a solvent containing the peptide, followed by stirring. 2-Nitropyridylsulfenylation can be carried out in a similar manner. The solvent for the reaction, reaction temperature, reaction time, etc. are substantially the same as described above [Peptide Chemistry, 1991, 125 (1992)].

1-1-B

A peptide having two thiol groups which are protected with appropriate protecting groups is elongated by a solid phase method. Before cleavage of the peptide from the resin, the thiol-protecting groups are selectively removed and the peptide is subjected to oxidation reaction to prepare a peptide having a cyclic structure. Then, the peptide is cleaved from the resin and the remaining protecting groups are removed, whereby the desired peptide having a cyclic structure is obtained.

Preferred examples of the thiol-protecting groups include an acetamidomethyl (Acm) group and a trityl (Trt) group. By reaction of the protected peptide on the resin with iodine in an appropriate solvent such as DMF or DCM, an Acm group and a Trt group are removed and an intramolecular disulfide bond is stimulaneously aformed. The reaction is carried out using 0.5-2 ml of a solvent for 50 mg of the resin, and iodine in an amount of 0.5-5 times, preferably one time the calculated weight of the peptide on the resin. The reaction is usually carried out at 0-40°C for one hour to one week. After the completion of reaction, the resin is subjected to a usual treatment in a solid phase method, that is, washing with a small amount of a solvent such as DMF or DCM, and then subjected to the subsequent reaction.

The pyridylsulfenylation or 2-nitropyridylsulfenylation of one of the SH groups as described in 1-A above can be applied to a solid phase method. The solvent for the cyclization reaction, reaction temperature, reaction time, etc. are substantially the same as described above. Further, similarly to the above-described liquid phase method, an equivalent amount of a reagent for pyridylsulfenylation or 2-nitropyridylsulfenylation may be introduced after the protecting groups of the two SH groups are removed. Pyridylsulfenylation can be carried out by adding 1-3 equivalents of a reagent such as 2,2'-dithiodipyridine to the resin swollen with a solvent, followed by stirring. 2-Nitropyridylsulfenylation can also be carried out in a similar manner. The solvent for the reaction, reaction temperature, reaction time, etc. are substantially the same as in the above cyclization reaction in the solid phase method.

(Process 1-2) Cyclization by S-$CH_2$-S bond formation

First a peptide having two thiol groups with S-dimethylphosphinothionyl groups introduced thereto is prepared by a solid phase method, a liquid phase method, or a combination thereof. Then, the peptide is subjected to reaction in the presence of tetrabutylammonium fluoride and methylene chloride, and the product is purified by general purification steps in organic chemical reactions to give the desired peptide having a cyclic structure [Peptide Chemistry 1995, edited by Norio Nishi, Protein Research Foundation, Osaka, p. 41 (1996)].

1-2-A

Any solvent which is inert to the reaction can be used. Preferred solvents are methylene chloride and solvent mixtures of methylene chloride and acetonitrile, tetrahydrofuran, chloroform, DMF, etc. The peptide concentration in the reaction mixture is 0.5-5000 μmol/l, preferably 50-500 μmol/l. Tetrabutylammonium fluoride is used in an amount of 2-10 equivalents, preferably 2-3 equivalents. The reaction is usually completed at 0-40°C for one hour to one week. After the completion of reaction, the peptide is precipitated by addition of diethyl ether, etc., followed by removal of the solvent. The product is purified by various kinds of chromatography, if necessary, and then can be subjected to the subsequent reaction. General purification steps in organic chemical reactions as described above are applicable as may be required.

1-2-B

A peptide having two thiol groups with S-dimethylphosphinothionyl groups introduced thereto is synthesized by a solid phase method. Before cleavage of the peptide from the resin, the peptide is subjected to reaction in the presence of tetrabutylammonium fluoride and methylene chloride to prepare a peptide having a cyclic structure. Then, the peptide is cleaved from the resin and the remaining protecting groups are removed, whereby the desired peptide having a cyclic structure is obtained.

By treatment of the protected peptide on the resin with tetrabutylammonium fluoride in a solvent such as DCM or a mixture of DCM and DMF, the desired cyclic structure is formed. This reaction is carried out using 0.5-2 ml of a solvent for 50 mg of the resin, and tetrabutylammonium fluoride in an amount of 2-20 equivalents based on the calculated molar quantity of the peptide on the resin. The reaction is usually carried out at 0-40°C for one hour to one week. After the completion of reaction, the resin is subjected to a usual treatment in a solid phase method, that is, washing with a small amount of a solvent such as DMF or DCM, and then can be subjected to the subsequent reaction.

(Process 1-3) Cyclization by S-$CH_2$-$C_6H_4$-$CH_2$-S bond formation

First a peptide having two thiol groups with S-benzyl groups introduced thereto is prepared by a solid phase method, a liquid phase method, or a combination thereof. Then, the peptide is subjected to reaction with metallic sodium in liquid ammonia in the presence of dibromoxylene, and the product is purified by general purification steps in organic chemical reactions to give the desired peptide having a cyclic structure [International Journal of Peptide and Protein Research, 40, 233 (1992)]. The desired peptide having a cyclic structure can also be prepared by first preparing a peptide which has, at two positions in the sequence, amino acid residue(s) and/or organic group(s) having a free thiol groups by a solid phase method, a liquid phase method, or a combination thereof, and then subjecting the peptide to reaction with dibromoxylene. -$C_6H_4$- (phenylene) in the cross-linkage structure includes o-phenylene, m-phenylene and p-phenylene.

Cyclization by -S-$(CH_2)$n-S- bond formation can be effected by using dibromoalkane instead of dibromoxylene in this process.

1-3-A

When a peptide having two thiol groups with S-benzyl groups introduced thereto is used, the peptide is dissolved in dry liquid ammonia and metallic sodium is added thereto until the blue coloration is maintained for several minutes, followed by addition of dibromoxylene to cause reaction. The peptide concentration in the reaction mixture is 0.5-

5000 μmol/l, preferably 50-500 μmol/l. Dibromoxylene is used in an amount of 1-3 equivalents, preferably 1-1.5 equivalents. The reaction is usually completed in 1-24 hours under reflux of liquid ammonia. After the completion of reaction, ammonia is removed and water is added to the residue, followed by purification by various kinds of chromatography as may be required. General purification steps in organic chemical reactions including such a step are applicable as may be required.

When a peptide having two free thiol groups is used, the peptide is dissolved in water containing 5-50%, preferably 10-20% methanol and 0.1% TFA or 1% acetic acid, and dibromoxylene is added thereto. The mixture is adjusted to pH 5-8, preferably pH 6.5-7.5 with dilute aqueous ammonia and the reaction is carried out. Dibromoxylene is used in an amount of 1-3 equivalents, preferably 1-1.5 equivalents. The reaction is usually completed at 0-40°C for one hour to one week. After the completion of reaction, the reaction mixture is purified as such, or after concentration under reduced pressure, by means of various kinds of chromatography. General purification steps in organic chemical reactions as described above are applicable as may be required.

1-3-B

A peptide having two thiol groups which are protected with appropriate protecting groups is elongated by a solid phase method. Before cleavage of the peptide from the resin, the thiol-protecting groups are selectively removed and the peptide is subjected to reaction with dibromoxylene in a solvent such as DMF or NMP to prepare a peptide having a cyclic structure. Then, the peptide is cleaved from the resin and the remaining protecting groups are removed, whereby the desired peptide having a cyclic structure is obtained.

By the reaction of the protected peptide on the resin with dibromoxylene in an appropriate solvent such as DMF or NMP, the cyclic structure moiety is formed. This reaction is completed using 0.5-2 ml of a solvent for 50 mg of the resin, and dibromoxylene in an amount of 1-3 equivalents based on the calculated molar quantity of the peptide on the resin. A base such as NMM may be added to the reaction mixture in an amount of 1-10 equivalents, preferably 2-5 equivalents. The reaction is usually carried out at 0-40°C for one hour to one week. After the completion of reaction, the resin is subjected to a usual treatment in a solid phase method, that is, washing with a small amount of a solvent such as DMF or DCM, and then can be subjected to the subsequent reaction.

(Process 1-4) Cyclization by S-$CH_2CO$- bond formation

First a peptide having an unprotected thiol group and a bromoacetyl group is prepared by a solid phase method, a liquid phase method, or a combination thereof. Bromoacetylation can be completed out according to an ordinary method of acetylating an amino group in a peptide chain. Then, the peptide is dissolved in water and the pH of the solution is adjusted to weakly basic to cause cyclization reaction. The reaction product is purified by general purification steps in organic chemical reactions to give the desired peptide having a cyclic structure [Journal of Medicinal Chemistry, 35, 2040 (1992)].

1-4-A

A peptide having an unprotected thiol group and a bromoacetyl group is dissolved in water. The solution is adjusted to pH 6-8, preferably pH 6.5-7.5 with dilute aqueous ammonia and the cyclization reaction is carried out. The peptide concentration in the reaction mixture is 0.5-5000 μmol/l, preferably 50-500 μmol/l. The reaction is usually carried out at 0-40°C for one hour to one week. After the completion of reaction, the reaction mixture is made weakly acidic by addition of acetic acid or the like, and then is purified as such, or after concentration under reduced pressure, by means of various kinds of chromatography. General purification steps in organic chemical reactions as described above are applicable as may be required.

1-4-B

A peptide having an appropriately protected thiol group and a bromoacetyl group is elongated by a solid phase method. Before cleavage of the peptide from the resin, the thiol-protecting group is selectively removed and the peptide is subjected to cyclization reaction by addition of an appropriate amount of an organic base such as NMM in a solvent such as DMF or NMP to prepare a peptide having a cyclic structure. Then, the peptide is cleaved from the resin and the remaining protecting groups are removed, whereby the desired peptide having a cyclic structure is obtained.

By the cyclization reaction of the protected peptide on the resin by addition of a base such as NMM in an appropriate solvent such as DMF or NMP, the desired cyclic structure is formed. This reaction is carried out using 0.5-2 ml of a solvent for 50 mg of the resin, and NMM is added in an amount of 1-10 equivalents, preferably 2-5 equivalents based on the calculated molar quantity of the peptide on the resin, followed by stirring. The reaction is usually carried out at 0-40°C for one hour to one week. After the completion of reaction, the resin is subjected to a usual treatment in a solid phase method, that is, washing with a small amount of a solvent such as DMF or DCM, and then can be subjected to the subsequent reaction.

(Process 2) Cyclization by CO-NH or NH-CO bond formation

First a peptide having an amino group and a carboxyl group each protected with an appropriate protecting group is prepared by a solid phase method, a liquid phase method, or a combination thereof. After the amino- and carboxyl-protecting groups are selectively removed, the peptide is subjected to intramolecular condensation, followed by general purification steps in organic chemical reactions to give a peptide having a cyclic structure. Then, the remaining protecting groups are removed, whereby the desired peptide is obtained. The desired peptide can also be prepared by first preparing a peptide moiety having a cyclic structure and then elongating it.
The cyclic structure may be formed by:

(1) cross-linkage between $X^p$ and $X^q$ in general formula (I); or
(2) a CO-NH bond between adjacent amino acids or organic acids which are to be constituents of the cyclic structure.

2-A

When a 4-methyltrityl group is used as the amino-protecting group, it can be removed by using acetic acid/trifluoroethanol/DCM (1/2/7). The reaction is usually carried out at 0-40°C for 0.5-6 hours. After the completion of reaction, the peptide is precipitated by addition of diethyl ether, etc., followed by removal of the solvent, on the solvent is removed under reduced pressure. General purification steps in organic chemical reactions including the above-described step are applicable as may be required.
When an allyloxycarbonyl group is used as the amino-protecting group and an allyl ester group is used as the carboxyl-protecting group, these protecting groups can be removed by reduction in the presence of a palladium catalyst. Any zerovalent palladium catalysts for homogenous system can be used in the reaction. Suitable catalysts include tetrakis(triphenylphosphine)palladium(0) and palladium( II) acetate-triphenylphosphine. The catalyst is used in an amount of 0.01-1 equivalent, preferably 0.1-0.5 equivalent, based on the above protecting groups. Further, additives such as formic acid, formic acid-triethylammonium, tributyltin hydride, triphenyltin hydride, trimethylhydrosilane, sodium borohydride, acetic acid, and acetic acid-NMM are added in an amount of one equivalent to excess based on the above protecting groups. As a solvent, ether, tetrahydrofuran, acetonitrile, DMF, chloroform, etc. can be used alone or in combination. For 1 mM of an allyloxycarbonyl group or allyl ester group are added the above reducing agent (a palladium catalyst and an additive) and 3-10 ml of the solvent. The reaction is carried out at -20 to 80°C, preferably 0 to 30°C for 10 minutes to 6 hours. After the completion of reaction, general purification steps in organic chemical reactions can be applied.
The above reaction is also applicable to a peptide in which only the carboxyl group is protected with an allyl ester group.
The obtained peptide is then subjected to reaction for forming an intermolecular amide bond between the amino group and the carboxyl group. Typical amidation reactions for cyclization are described below. Common reaction conditions are as follows. As a solvent, DMF, NMP, methylene chloride, chloroform, acetonitrile, tetrahydrofuran, etc. are used alone or in combination. The peptide is used at a concentration of 0.5-5000 μmol/l, preferably 50-500 μmol/l. The reaction is carried out usually at 0-40°C, preferably 4-25°C, with stirring for 3 hours to one week. After the completion of reaction, general purification steps in organic chemical reactions can be applied.
Amidation reaction can be carried out by using carbodiimide such as dicyclohexylcarbodiimide (DCC) or water-soluble carbodiimide (WSC) in an amount of 1-10 equivalents based on the carboxyl group. NMM, DIEA or sodium hydrogen carbonate is added in an amount of 1.5-2 equivalents based on carbodiimide. If necessary, HOBt or HONSu may be added as an additive in an equimolecular amount based on carbodiimide.
Amidation reaction can also be carried out by using a condensing agent such as diphenylphosphoryl azide (DPPA) or diethyl phosphorocyanidate (DEPC) in an amount of 1-10 equivalents based on the carboxyl group. NMM, DIEA or sodium hydrogen carbonate is added as an additive in an amount of 1.5-2 equivalents based on the condensing agent.
Further, amidation reaction can be carried out by using a condensing agent such as PyBOP, HBTU or HATU in an amount of 1-10 equivalents, preferably 2-5 equivalents based on the carboxyl group, and HOBt in an equimolecular amount based on the condensing agent. HATU may be used alone without addition of HOBt. NMM, DIEA or sodium hydrogen carbonate is added in an amount of 1.5-2 equivalents based on the condensing agent.
It is also possible to convert the carboxyl group into an active ester, selectively remove the amino-protecting

group, and then form an amide bond. Examples of the active esters are p-nitrophenyl ester, pentafluorophenyl ester, and N-oxysuccinimido ester. The active esters can be formed by ordinary methods. For example, DCC is added in an amount of 1-10 equivalents based on the carboxyl group, together with an equimolecular amount of p-nitrophenol, pentafluorophenol or HONSu, and the mixture is stirred at 0-5°C for one hour to one day. The solvent for the formation of active esters, reaction temperature, reaction time, etc. are substantially the same as in the above amidation reactions.

2-B

By a solid phase method, a peptide is synthesized which has an amino acid residue having an amino group protected with an appropriate protecting group and a carboxyl group protected with an appropriate protecting group. Before cleavage of the peptide from the resin, the amino- and carboxyl-protecting groups are selectively removed, and the resulting amino group and carboxyl group are subjected to condensation reaction to give a peptide moiety having a cyclic structure. Then, the peptide is cleaved from the resin and the remaining side-chain-protecting groups are removed, whereby the desired peptide having a cyclic structure is obtained.

When a 4-methyltrityl group is used as the amino-protecting group, it can be removed by reaction using 0.5-2 ml of acetic acid/trifluoroethanol/DCM (1/2/7) for 50 mg of the resin. The reaction is usually carried out at 0-40°C for 0.5-6 hours. After the completion of reaction, the resin is subjected to a usual treatment in a solid phase method, that is, washing with a small amount of a solvent such as DMF, and then can be subjected to the subsequent reaction.

When an allyloxycarbonyl group is used as the amino-protecting group and an allyl ester group is used as the carboxyl-protecting group, these protecting groups can be removed by reaction using, for example, a chloroform solution containing 0.1-0.2 M tetrakis(triphenylphosphine)palladium(0), 5% acetic acid and 2.5% NMM. For 1 mM of an allyloxycarbonyl group and an allyl ester group is added 3-10 ml of the above chloroform solution. The reaction is usually carried out at 0-40°C for 0.5-6 hours. After the completion of reaction, the resin is subjected to a usual treatment in a solid phase method, that is, washing with a small amount of a solvent such as DMF, and then can be subjected to the subsequent reaction.

The subsequent reaction is carried out by using an organic solvent such as DMF, DCM or NMP containing a condensing agent such as PyBOP or HBTU in an amount of 1-10 equivalents, preferably 2-5 equivalents based on the calculated quantity of the carboxyl group on the resin, HOBt in an equimolecular amount based on the condensing agent if necessary, and NMM or DIEA in an amount of 1.5-2 equivalents based on the condensing agent. For 50 mg of the resin is used 1 ml of the solvent. The reaction is carried out usually at 0-40°C, preferably 4-25°C, with stirring for 3 hours to one week. After the completion of reaction, the resin is subjected to a usual treatment in a solid phase method, that is, washing with a small amount of a solvent such as DMF, and then can be subjected to the subsequent reaction.

(Process 3) Cyclization by CO-O or O-CO bond formation

First a peptide having a hydroxy group and a carboxyl group each protected with an appropriate protecting group is prepared by a solid phase method, a liquid phase method, or a combination thereof. After the hydroxy- and carboxyl-protecting groups are selectively removed, the peptide is subjected to condensation, followed by general purification steps in organic chemical reactions to give a peptide having a cyclic structure. Then, the remaining protecting groups are removed, whereby the desired peptide is obtained. The desired peptide can also be prepared by first preparing a peptide moiety having a cyclic structure and then elongating it.

3-A

When a trityl group is used as the hydroxy-protecting group, it can be removed by reaction using TFA/TFE/DCM (5/5/90 - 1/5/94). The reaction is usually carried out at 0-40°C for 0.5-12 hours. After the completion of reaction, the product can be purified by general purification steps in organic chemical reactions. A p-methoxybenzyl group and a 2,4-dimethoxybenzyl group can also be used as the selective hydroxy-protecting group, and they can be removed by reaction using 2,3-dichloro-5,6-dicyano-1,4-benzoquinone or ceric ammonium nitrate in an amount of 1-10 equivalents based on the protected hydroxy group. As a solvent, DMF, NMP, methylene chloride, chloroform, acetonitrile, tetrahydrofuran, etc. which contain water can be used alone or in combination. The reaction is usually carried out at 0-40°C for 0.5-12 hours. After the completion of reaction, the product can be purified by general purification steps in organic chemical reactions.

Selective protection of the desired carboxyl group and removal of the protecting group can be carried out according to the procedure in Process 2, 2-A.

The obtained peptide is then subjected to reaction for forming an intramolecular ester bond between the hydroxy group and the carboxyl group. Typical esterification reactions for cyclization are described below. Common reaction conditions are as follows. As a solvent, DMF, NMP, methylene chloride, chloroform, acetonitrile, tetrahydrofuran, etc. are used alone or in combination. The peptide is used at a concentration of 0.5-5000 μmol/l, preferably 50-500 μmol/l. The reaction is carried out usually at -20 to 40°C, preferably -20 to 30°C, with stirring for 3 hours to one week. After the completion of reaction, general purification steps in organic chemical reactions can be applied.

(1) Esterification reaction can be carried out by adding 1-10 equivalents of DCC to a solution of a peptide having the hydroxy group at the side chain and stirring the mixture at -20 to 40°C, preferably -20 to 20°C. It is preferable to use pyridine as a solvent or in a solvent at a concentration of 10-100%.
(2) Esterification reaction can be carried out by adding 1-(2-mesitylenesulfonyl)-3-nitro-1,2,4-triazole (MSNT) in an amount of 1-5 equivalents based on the carboxyl group, and 0.5-1.5 equivalents of N-methylimidazole, and stirring the mixture [Tetrahedron Letters, p. 1701 (1990)].
(3) Esterification reaction can also be carried out by using DEPC in an amount of 1-10 equivalents, preferably 2-5 equivalents based on the carboxyl group [Tetrahedron Letters, p. 1595 (1973)].

3-B

A peptide having a hydroxy group and a carboxyl group each protected with an appropriate protecting group is synthesized by a solid phase method. Before cleavage of the peptide from the resin, the hydroxy- and carboxyl-protecting groups are selectively removed, and the resulting hydroxy group and carboxyl group are subjected to condensation reaction to form the desired cyclic ester moiety. Then, the peptide is cleaved from the resin and the remaining protecting groups are removed, whereby the desired peptide having a cyclic structure is obtained.
When a trityl group is used as the hydroxy-protecting group, it can be removed by reaction using 0.5-2 ml of TFA/TFE/DCM (5/5/90 - 1/5/94) for 50 mg of the resin. The reaction is usually carried out at 0-40°C for 0.5-12 hours. A p-methoxybenzyl group and a 2,4-dimethoxybenzyl group can also be used as the hydroxy-protecting group, and they can be removed by reaction using 2,3-dichloro-5,6-dicyano-1,4-benzoquinone or ceric ammonium nitrate in an amount of 1-10 equivalents based on the hydroxy-protecting group. As a solvent, an organic solvent (e.g. DMF, DCM or NMP) which contains water is used in an amount of 0.5-1 ml for 50 mg of the resin. The reaction is carried out usually at 0-40°C, preferably 0-25°C, for 0.5-12 hours. After the completion of reaction, the resin is subjected to a usual treatment in a solid phase method, that is, washing with a small amount of a solvent such as DMF, and then can be subjected to the subsequent reaction.
Selective protection of the desired carboxyl group and removal of the protecting group can be carried out according to the procedure in Process 2, 2-B.
The subsequent reaction is carried out by using, for 50 mg of the resin, 0.5-2 ml of DMF or NMP containing a condensing agent such as MSNT or DEPC in an amount of 1-10 equivalents, preferably 2-5 equivalents based on the calculated quantity of the carboxyl group on the resin. The reaction is carried out usually at -20 to 40°C, preferably -20 to 20°C, with stirring for 3 hours to one week. After the completion of reaction, the resin is subjected to a usual treatment in a solid phase method, that is, washing with a small amount of a solvent such as DMF, and then can be subjected to the subsequent reaction.
Compounds (I) obtained by the above processes can be purified by high performance liquid chromatography (hereinafter referred to as HPLC) using various kinds of reversed-phase silica gel columns such as C-4, C-8 or C-18, partition column chromatography, various kinds of column chromatography or thin layer chromatography using adsorption resins, silica gel, chemically-modified silica gel, reversed-phase silica gel, alumina, diatomaceous earth, magnesium silicate or ion-exchange resins, or gel filtration using various kinds of Sepharose or Sephadex.
The pharmaceutically acceptable salts of Compound (I) are obtained according to an ordinary method. That is, the acid addition salts and organic base addition salts of Compound (I) are obtained by dissolving Compound (I) in an aqueous solution of the corresponding acid or organic base, followed by lyophilization or purification in the same manner as above. The metal salts of Compound (I) are obtained by dissolving Compound (I) in an aqueous solution containing the corresponding metal ion, followed by purification in the same manner as above.
The pharmacological activities of representative Compounds (I) are described below by Test Examples.

<u>Test Example 1</u> Measurement of the Activity of Restoring the DNA-Binding to Mutant P53 Using Purified P53 Protein

<u>Construction of Recombinant Baculovirus for Expression of Wild-Type and Mutant P53</u>

In order to measure the DNA-binding activity of mutant P53, human wild-type P53 and two kinds of human P53 mutants were expressed in insect cells using a baculovirus in the following manner. For the production of a protein in insect cells, it is necessary to construct a recombinant virus carrying the desired gene. The construction process comprises the step of constructing a transfer vector by inserting cDNA coding for the desired protein into a specific plasmid and the step of obtaining a recombinant virus through homologous recombination caused by cotransfection of a wild-type virus and the transfer vector in an insect cell. These steps were carried out using BaculoGold Starter Kit (Pharmingen, product No. PM-21001K) according to its manual as described below.

NcoI-XbaI fragments (1.6 kb) containing the complete p53 translational region were isolated from plasmids WT393, 273His and 248Trp which respectively carry human wild-type p53 cDNA and two kinds of its mutants (Arg273→His and Arg248→Trp) [Proceedings of the National Academy of Science USA, 89, 5403 (1992)]. The obtained fragments were respectively inserted into a XbaI site of baculovirus expression vector pVL1393 (Pharmingen) via the sequences having XbaI and NcoI cleavage sites at ends shown below (SEQ ID NOS: 8 and 9) to construct transfer vectors pBP53WT, pBP53M7 and pBP53M6. The above mutations (Arg273→His and Arg248→Trp) are both mutations of P53 commonly found in human cancers. The mutant proteins are hereinafter referred to as P53His273 and P53Trp248, respectively.

(SEQ ID NOS: 8 and 9)

5'-CTAGACAGCCAGACTGCCTTCCGGGTCACTGC-3'
3'-TGTCGGTCTGACGGAAGGCCCAGTGACGGTAC-5'

Construction of the recombinant baculovirus was carried out by introducing filamentous baculovirus DNA (BaculoGold baculovirus DNA, Pharmingen) and the transfer vector DNA into insect cells Sf9 (Pharmingen, hereinafter referred to as Sf9 cells) cultured in TMN-FH insect medium (Pharmingen, hereinafter referred to as TMN-FH medium) by the lipofectin method [Protein, Nucleic Acid, Enzyme, 37,2701 (1992)] in the following manner.

Each of the transfer vectors pBP53WT, pBP53M7 and pBP53M6 (1 μg) and 20 ng of the filamentous baculovirus DNA were dissolved in 12 μl of distilled water, followed by addition of a mixture of 6 μl of lipofectin and 6 μl of distilled water. The resulting mixture was allowed to stand at room temperature for 15 minutes. Separately, Sf9 cells (2 x $10^6$ cells) were suspended in 2 ml of Sf900-II insect medium (Gibco, hereinafter referred to as Sf900-II medium) and the suspension was put into a plastic Dish for culturing (diameter: 35 mm). To the Dish for culturing cells was added the whole of the above solution containing the plasmid DNA, the filamentous baculovirus DNA and lipofectin. After culturing at 27°C for 3 days, 1 ml of the culture supernatant containing the recombinant virus was separated. Then, 1 ml of fresh Sf900-II medium was added to the Dish for culturing cells, followed by culturing at 27°C for 3 days to further obtain 1.5 ml of the culture supernatant containing the recombinant virus.

The obtained recombinant virus was proliferated in the following manner to be used for the expression of a protein.

Sf9 cells (2 x $10^7$ cells) were suspended in 10 ml of Sf900-II medium and the suspension was allowed to stand at room temperature for one hour in a flask (175 $cm^2$, Gliner) to attach the cells thereto. After the culture supernatant was removed, 15 ml of TMN-FH medium and 1 ml of the above supernatant containing the recombinant virus were added to the flask, followed by culturing at 27°C for 3 days. The culture supernatant was centrifuged at 1500 x g for 10 minutes to remove the cells, whereby a solution of the recombinant virus to be used for the expression of a protein was obtained.

The obtained recombinant virus solution was examined for virus titer in the following manner (Pharmingen, BaculoGold Starter Kit Manual). Sf9 cells (6 x $10^6$ cells) were suspended in 4 ml of Sf900-II medium and the suspension was allowed to stand at room temperature for one hour in a plastic Dish for culturing cells (diameter: 60mm) to attach the cells thereto. After the supernatant was removed, 400 μl of Sf900-II medium and the above recombinant virus solution diluted 10000-fold with Sf900-II medium were added to the dish. The mixture was allowed to stand at room temperature for one hour, and then the medium was removed, followed by addition of 5 ml of a medium containing 1% low-gelling-temperature agarose (Agarplaque Agarose, Pharmingen) (a mixture of 1 ml of sterilized 5% aqueous solution of Agarplaque Agarose and 4 ml of TMN-FH medium which had been kept at 42°C). After the mixture was allowed to stand at room temperature for 15 minutes, the dish was wound with a plastic tape to avoid drying and then sealed into a plastic vessel. Culturing was carried out at 27°C for 6 days, and 1 ml of phosphate buffered saline (137 mM NaCl, 2.7mM KCl, 8.1mM $Na_2HPO_4$, 1.5mM $KH_2PO_4$; hereinafter referred to as PBS buffer) containing 0.01% Neutral Red was added to the dish. After culturing for further one day, the number of plaques formed was counted. In this manner, the recombinant virus solutions obtained above were each found to contain the virus of about 1 x $10^8$ plaque-forming unit (hereinafter referred to as pfu/ml).

Expression of Wild-type and Mutant P53 in Insect Cells

According to the manual attached to BaculoGold Starter Kit (Pharmingen), wild-type P53 and mutants P53His273 and P53Trp248 were expressed in the following manner. Recovery of each protein from the culture was carried out by a modification of the method of D. A. Daniels, et al. [Journal of Molecular Biology, 243, 639 (1994)] using Heparin-Sepharose (both by Pharmacia Biotech).

Wild-type and mutant P53 proteins were obtained in the following manner. Sf9 cells (4 x $10^7$ cells) which had been cultured in advance at 27°C were suspended in 30 ml of Sf900-II medium in a flask (175 $cm^2$, Gliner) and the suspension was allowed to stand at 27°C for 30 minutes, followed by removal of the culture supernatant. To the flask were added 10 ml of Sf900-II medium and 2 ml of the above solution containing the recombinant virus derived from one of the transfer vectors pBP53WT, pBP53M7 and pBP53M6 at a concentration of about 1 x $10^8$ pfu/ml, and the mixture was slowly shaken at room temperature for 1-2 hours. Then, 20 ml of TMN-FH medium was added, followed by culturing at 27°C for 3-4 days. After the completion of culturing, the cells were collected by vigorous shaking and centrifuged at 1500 x g for 10 minutes to obtain a precipitate. The precipitate was washed twice with 5 ml of PBS buffer and the cells in which wild-type or mutant P53 protein was expressed were collected as a precipitate.

The obtained cells in which wild-type or mutant P53 was expressed (3.6 x $10^8$ cells) were suspended in 10 ml of a buffer [10 mM Tris-HCl (pH 7.5), 10 mM KCl, 2 mM $MgCl_2$, 1 mM dithiothreitol (hereinafter referred to as DTT), 0.5 mg/ml ethylenediaminetetraacetic acid (hereinafter referred to as EDTA), 1 mg/ml benzamidine, 10 μg/ml leupeptin and 10 μg/ml antipain; hereinafter referred to as G buffer] and the suspension was layered over 10 ml of G buffer containing 0.5 M sucrose in a 50-ml centrifuge tube (Falcon, 2070). Centrifugation was carried out at 1500 x g at 4°C for 10 minutes and the obtained precipitate was suspended in 5 ml of G buffer. The suspension was put into a Teflon homogenizer (Iwaki Glass Co., Ltd.) and the pestle was moved up and down 30 times to disrupt the cells. Then, 5 ml of the disrupted cell suspension was layered over 5 ml of G buffer containing 0.5 M sucrose in a 50-ml centrifuge tube, followed by centrifugation at 1500 x g at 4°C for 10 minutes. The precipitated nuclear fraction was collected and suspended in 5 ml of a dissolution buffer [150 mM NaCl, 50 mM Tris-HCl (pH 8.0), 5 mM EDTA, 1% Nonidet P-40 (hereinafter referred to as NP-40) and 1 mM benzamidine; hereinafter referred to as dissolution buffer 1]. After being allowed to stand at 0°C for 40 minutes, the suspension was centrifuged at 12000 x g at 4°C for 30 minutes. The obtained supernatant was diluted four-fold with a buffer [20 mM 4-(2-hydroxyethyl)-1-piperazineethanesulfonic acid (hereinafter referred to as HEPES)-KOH (pH 8.0), 0.1 mM EDTA, 0.1% Triton X 100, 5mM DTT, 1 mM benzamidine and 15% glycerol; hereinafter referred to as A buffer] and then apllied to a column of Hi-Trap™ Heparin-Sepharose (5 ml, Pharmacia Biotech) pre-equilibrated with A buffer. After the column was washed with 50 ml of A buffer containing 0.1 M KCl, 40 ml (total volume) of A buffer with linear KCl concentration gradient of 0.1-0.6 M was apllied to the column to elute a protein bound to Heparin. The eluate was taken in 2 ml fractions and each fraction was analyzed by SDS-polyacrylamide electrophoresis, whereby 8 ml each of fractions respectively containing wild-type P53, mutant P53His273 and mutant P53Trp248 proteins at high concentrations were obtained. The concentrations of proteins as determined with a densitometer were as follows: wild-type, 212 μg/ml; mutant P53His273, 230 μg/ml; mutant P53Trp248, 20 μg/ml.

Gel Shift Assay Using Wild-type and Mutant P53 Proteins

Two oligonucleotides (SEQ ID NOS: 10 and 11) comprising complementary sequences which have fluorescence-labelled 5'-end and have P53-binding sequence [Nature Genetics, 1, 45 (1992)] in the center part were synthesized using FluorePrime™ (Pharmacia Biotech) and a DNA synthesizer (ABI, Model 392).

(SEQ ID NOS: 10 and 11)

5'-TCGAGAGACATGCCTAGACATGCCTG-3'
5'-TCGACAGGCATGTCTAGGCATGTCTC-3'

The above two oligonucleotides (200 pmol each) were dissolved in 0.2 ml of a buffer [10 mM Tris-HCl (pH 7.5) and 1 mM EDTA; hereinafter referred to as TE buffer] and the solution was kept at 70°C for 10 minutes and then slowly cooled to room temperature to cause annealing. The annealing product was used as fluorescence-labelled P53 probe.

To the following substances was added distilled water to make a total volume of 20 μl, and the mixture was subjected to reaction at 4°C for 30 minutes.

1) 0.2 pmol of fluorescence-labelled P53 probe
2) 100 ng of wild-type P53 protein or mutant P53 protein (P53His273 or P53Trp248) expressed in Sf9 cells
3) 2 μg of calf thymus DNA
4) 20 mM HEPES (pH 7.4), 40 mM KCl, 1 mM $MgCl_2$, 0.1 mM EDTA, 0.1% NP-40, 1 mM DTT
5) 0-100 μM test compound

After the completion of reaction, the reaction mixture was subjected to 4% polyacrylamide gel electrophoresis using 0.5xTBE [2.5 mM Tris-Borate (pH 8.3) and 0.1 mM EDTA] as the electrophoresis buffer. As the wild-type P53 protein binds to the fluorescence-labelled P53 probe, its electrophoretic mobility is decreased and the protein is detected as a band in the upper part. On the other hand, both of two mutant P53 proteins per se are not capable of binding to the probe; however, the addition of a test compound restores the DNA-binding activity to them and enables detection of shifting bands. After the electrophoresis, detection of fluorescent bands was carried, out using FluorImager™ SI fluorescence image analyzer (Molecular Dynamics). The results on the mutant P53His273 and the mutant P53Trp248 are shown in Figs. 1 and 2, respectively.

The amount of fluorescent substance in the shifting band in each lane was measured with FluorImager™ SI fluorescence image analyzer, and the rate of activation was calculated according to the following equation using the sample which contains no test compound in the reaction system as a control. Although in cases where the mutant P53 proteins are used, shifting bands may not be clearly identified in the absence of a test compound, the amount of fluorescent substance contained in the region corresponding, in location and size, to the band observed in the presence of a test compound was measured. The results are shown in Tables 2-1 and 2-2.

$$\text{Rate of Activation} = B/A$$

A: Amount of fluorescent substance in the shifting band in the absence of a test compound
B: Amount of fluorescent substance in the shifting band in the presence of a test compound

Table 2-1

| Rate of Activation | | |
|---|---|---|
| Compound | Rate of Activation (%) | |
| | P53His273 | P53Trp248 |
| Compound 1 | 8.0 | 2.2 |
| Compound 2 | 10.3 | 1.2 |
| Compound 3 | 3.3 | 1.8 |
| Compound 4 | 37.2 | 6.0 |
| Compound 5 | 15.0 | 3.5 |
| Compound 6 | 22.5 | 11.2 |
| Compound 7 | 12.4 | 4.4 |
| Concentration of test compound: 100 µM | | |

Table 2-2

| Rate of Activation | | |
|---|---|---|
| Compound | Rate of Activation (%) | |
| | P53His273 | P53Trp248 |
| Compound 8 | 5.6 | unmeasured |
| Compound 11 | 4.1 | 4.4 |
| Compound 17 | 5.6 | unmeasured |
| Compound 18 | 8.7 | 2.8 |
| Compound 19 | 9.4 | 1.6 |
| Compound 20 | 6.8 | 3.3 |
| Compound 21 | 5.4 | 3.8 |
| Compound 22 | 7.0 | 3.4 |

Table 2-2 (continued)

| Rate of Activation | | |
|---|---|---|
| Compound | Rate of Activation (%) | |
| | P53His273 | P53Trp248 |
| Compound 23 | 10.7 | 5.0 |
| Compound 24 | 11.8 | 1.9 |
| Concentration of test compound: 100 μM | | |

Test Example 2 Measurement of the Activity of Restoring the DNA-Binding to Mutant P53 Using Cultured Cell Extract

It has been reported that the p53 gene in human epidermoid carcinoma cell A431 (American Type Culture Collection, ATCC CRL-1555) has a mutation of Arg→His at codon 273 [Cancer Research, 54, 2834 (1994)]. Gel shift assay using the nuclear extract prepared from A431 cells has revealed that the test compounds are capable of restoring the DNA-binding activity not only to recombinant mutant P53 proteins but also to mutant P53 protein expressed in cells as shown below.

Preparation of Nuclear Extract from A431 Cells

Human A431 cells were suspended in 10 ml of Dulbecco's Modified Eagle medium Nissui (Nissui Pharmaceutical Co., Ltd.) containing 10% fetal bovine serum (FBS)(hereinafter referred to as DMEM-FBS medium) to a concentration of 1-2 x $10^5$ cells/ml, and 10 ml of the suspension was poured into a dish for culturing cells (diameter: 10 cm, Iwaki Glass Co., Ltd.) Culturing was carried out at 37°C for 2 days in the presence of 5% $CO_2$, and the medium was removed with suction, followed by washing with 5 ml of PBS buffer. After 2 ml of PBS buffer was added, the cells were scraped off using a scraper and put into a 1.5-ml micro centrifuge tube. Centrifugation was carried out at 1000 x g at 4°C for 10 minutes, and the obtained precipitate was suspended in 0.4 ml of a buffer [20 mM HEPES (pH 7.9), 50 mM NaCl, 1 mM EDTA, 10% glycerol, 0.1% NP-40, 1 mM DTT, 1 mM phenylmethylsulfonyl fluoride (hereinafter referred to as PMSF), 1 mM $Na_3VO_4$ and 1 mg/ml leupeptin]. The suspension was allowed to stand at 0°C for 10 minutes and then centrifuged at 1000 x g at 4°C for 10 minutes to obtain the nuclear fraction as a precipitate. The precipitate was suspended in 0.4 ml of a dissolution buffer [20 mM HEPES (pH 7.9), 50 mM NaCl, 10 mM EDTA, 2 mM ethylenebis(oxyethylenenitrilo)tetraacetic acid (hereinafter referred to as EGTA), 0.1% NP-40, 1 mM PMSF, 1 mM $Na_3VO_4$ and 1 mg/ml leupeptin; hereinafter referred to as dissolution buffer 2], followed by vigorous shaking at 4°C for 10-20 minutes. Then, the suspension was centrifuged at 10000 x g at 4°C for 10 minutes to obtain a crude nuclear extract as a supernatant. The protein concentration in the extract was determined by using a protein assay kit (BIO-RAD). By the above procedure, 0.4 ml of a protein solution (3.5 mg/ml) was obtained.

Gel Shift Assay Using A431 Cell Extract

Gel shift assay was carried out using the obtained A431 cell nuclear extract and the same fluorescent P53-binding sequence DNA probe as used above. To the following substances was added distilled water to make a total volume of 20 μl, and the mixture was subjected to reaction at 4°C for 30 minutes.

1) 0.2 pmol of fluorescence-labelled P53 probe
2) 35 μg of A431 cell nuclear extract (on the basis of the amount of protein)
3) 2 μg of poly(dI-dC) • poly(dI-dC) (Pharmacia Biotech)
4) 20 mM HEPES (pH 7.4), 40 mM KCl, 1 mM $MgCl_2$, 0.1 mM EDTA, 0.1% NP-40, 1 mM DTT
5) 0-100 μM test compound

After the completion of reaction, the reaction mixture was subjected to 4% polyacrylamide gel electrophoresis using 0.5xTBE [2.5 mM Tris-Borate (pH 8.3) and 0.1 mM EDTA] as the electrophoresis buffer. As the P53 protein in A431 cells is a mutant protein (273Arg→His), it is not capable of binding to the probe; however, the addition of a test compound restores the DNA-binding activity to it and enables detection of a shifting band. After the electrophoresis, detection of fluorescent bands was carried out using FluorImager™ SI fluorescence image analyzer (Molecular Dynamics). The results are shown in Fig. 3. The amount of fluorescent substance in the shifting band in each lane was measured with FluorImager™ SI fluorescence image analyzer, and the rate of activation was calculated according to the following equation using on the sample which contains no test compound in the reaction system as a control. Although

shifting bands may not be clearly identified in the absence of a test compound, the amount of fluorescent substance contained in the region corresponding, in location and size, to the band observed in the presence of a test compound was measured. The results are shown in Tables 3-1 and 3-2.

$$\text{Rate of Activation} = B/A$$

A: Amount of fluorescent substance in the shifting band in the absence of a test compound
B: Amount of fluorescent substance in the shifting band in the presence of a test compound

Table 3-1

| Compound | Rate of Activation (%) |
|---|---|
| Compound 1 | 3.3 |
| Compound 2 | 4.6 |
| Compound 3 | 3.7 |
| Compound 4 | 5.2 |
| Compound 5 | 5.3 |
| Compound 6 | 3.3 |
| Compound 7 | 3.4 |
| Concentration of test compound: 100 μM | |

Table 3-2

| Compound | Rate of Activation (%) |
|---|---|
| Compound 8 | 4.0 |
| Compound 11 | 3.8 |
| Compound 17 | 3.0 |
| Compound 18 | 3.4 |
| Compound 19 | 5.8 |
| Compound 20 | 4.6 |
| Compound 21 | 2.9 |
| Compound 22 | 5.7 |
| Compound 23 | 3.8 |
| Compound 24 | 2.5 |
| Concentration of test compound: 100 μM | |

<u>Test Example 3</u> Measurement of the Activity of Activating P53-Dependent Transcription

It was confirmed by the following experiment using luciferase reporter that the introduction of a test compound into a cell having mutated p53 gene causes restoration of the transcription activity to mutant P53 to enhance P53-dependent transcription.

<u>Construction of P53 Responding Reporter Plasmid</u>

Plasmid p53RE8luc2 comprising 8 copies of the P53 responsive element in the transcription regulatory region

and the firefly luciferase gene downstream therefrom as a reporter gene was constructed according to the following procedure. An outline of the construction steps is illustrated in Fig. 4.

A luciferase reporter vector pSE01uc2 for the insertion of the transcription regulatory region, was constructed by inserting at the XhoI and HindIII cleavage sites of luciferase reporter vector pluc2 containing the neomycin-resistance gene [European Journal of Haematology, 52, 73 (1994)] (1) an SphI-HindIII fragment consisting of 200 base pairs derived from the core promoter region of SV40 early gene [128-0/5243-5171; the base numbers are indicated in accordance with Molecular Biology of Tumor Viruses, 2nd Ed., Part 2 Revised, DNA Tumor Viruses, edited by J. Tooze, Cold Spring Harbor Laboratory Press (1982)] and (2) a fragment which is obtained by annealing of the two synthetic oligonucleotides shown below (SEQ ID NOS: 12 and 13) and which has XhoI and SphI sites at both ends and SmaI and Asp718 (KpnI) sites within the sequence.

(SEQ ID NOS: 12 and 13)

5'-TCGAGCCCGGGGGTACCGCATG-3'
5'-CGGTACCCCCGGGC-3'

Subsequently, reporter plasmid p53RE1luc2 comprising one copy of the P53 responsive element in the transcription regulatory region was constructed by annealing the two complementary oligonucleotides shown below (SEQ ID NOS: 14 and 15) which contain the P53 responsive element and inserting the annealing product at the XhoI and Asp718 sites of the luciferase vector pSE0luc2 obtained above.

(SEQ ID NOS: 14 and 15)

5'-TCGAGGGACTTGCCTGGACTTGCCTGTCGACG-3'
5'-GTACCGTCGACAGGCAAGTCCAGGCAAGTCCC-3'

P53RE11uc2 thus obtained was cleaved with restriction enzymes XhoI and MluI simultaneously, followed by agarose gel electrophoresis to separate a 5.3 kb XhoI-MluI DNA fragment. Similarly, p53RE1luc2 was cleaved with SalI and MluI simultaneously to obtain a 2.6 kb MluI-SalI DNA fragment. Ligation of both fragments gave reporter plasmid p53RE2luc2 comprising 2 copies of the P53 responsive element (see Fig. 5).

Then, p53RE2luc2 was cleaved with restriction enzymes XhoI and MluI simultaneously, followed by agarose gel electrophoresis to separate a 5.3 kb XhoI-MluI DNA fragment. Similarly, p53RE2luc2 was cleaved with SalI and MluI simultaneously to obtain a 2.6 kb MluI-SalI DNA fragment. Ligation of both fragments gave reporter plasmid p53RE4luc2 comprising 4 copies of the P53 responsive element (see Fig. 6).

The same procedure as above was repeated using obtained p53RE4luc2 to obtain reporter plasmid p53RE8luc2 comprising 8 copies of the P53 responsive element (see Fig. 7).

Introduction of DNA and Test Compounds into Cells

The test compounds were introduced into cells using the liposome reagent for DNA transfection DOSPER™ (Boehringer Mannheim GmbH).

Human epidermoid carcinoma A431 cells (American Type Culture Collection, ATCC CRL-1555) were put into wells of a 6-well dish for culturing cells (diameter: 35 mm, Iwaki Glass Co., Ltd.) at a cell density of 4 x $10^5$ cells/well and cultured in DMEM-FBS medium at 37°C for 24 hours in the presence of 5% $CO_2$. A test compound (50 nmol) and 2 μg of reporter plasmid p53RE8luc2 were dissolved in a buffer (20 mM HEPES and 150 mM NaCl, pH 7.4; hereinafter referred to as HBS buffer) to make 100 μl of a solution (Solution A). Separately, 5 μl of a Dosper solution was added to HBS buffer to make 100 μl of a solution (Solution B). Solutions A and B were gently mixed and the mixture was allowed to stand at room temperature for 15 minutes, followed by addition of 0.8 ml of Opti-MEM$^R$I medium (Gibco BRL).

From the culture of A431 cells cultured in the above 6-well dish, the medium was removed with suction. After the wells were washed with 2 ml of Opti-MEM$^R$I medium (Gibco BRL), 1 ml of the mixture of Solutions A and B prepared above was gently added onto the cells. Culturing was carried out at 37 °C for 6 hours in the presence of 5% $CO_2$, and 1 ml of DMEM-FBS medium was added, followed by further culturing for 24 hours. The final concentration of the test compound was 25 μM.

Measurement of the Activity of Activating P53-Dependent Transcription

After the culturing for 24 hours, the above cells were scraped off using a scraper and collected by centrifugation. The collected cells were suspended in 0.5 ml of a dissolution buffer [1% Triton X 100, 100 mM $KH_2PO_4$ (pH 7.8) and 1 mM DTT] and the suspension was centrifuged to obtain a supernatant. Then, 200 µl of the supernatant was subjected to measurement of luciferase activity, and 10 µl of the supernatant was subjected to measurement of protein concentration. The luciferase activity was determined by the use of Luminometer LB953 (Beltold), as the amount of luminescence measured for 10 seconds after automatic addition of 300 µl of a substrate solution [25 mM glycylglycine (pH 7.8), 15 mM $MgSO_4$, 5 mM ATP and 0.33 mM luciferin]. The protein concentration was determined by using BIO-RAD Protein Assay Kit (BIO-RAD), and the obtained value was used for normalization of the luciferase activity. The rate of activation was calculated according to the following equation using the luciferase activity (normalized with the protein concentration) of the sample which contains no test compound in the reaction system as a control. The results are shown in Table 4.

Table 4

| Compound | Rate of Activation (%) |
|---|---|
| Compound 1 | 1.4 |
| Compound 4 | 6.3 |
| Compound 8 | 3.9 |
| Compound 23 | 5.0 |

$$\text{Rate of Activation} = B/A$$

A: Luciferase activity in the absence of a test compound (normalized with protein concentration)
B: Luciferase activity in the presence of a test compound (normalized with protein concentration)

Test Example 4 Measurement of Growth Inhibiting Activity against A431 Cells

Human epidermoid carcinoma A431 cells (1 x $10^6$ cells) cultured in DMEM-FBS medium at 37°C in the presence of 5% $CO_2$ were suspended in 50 µl of a buffer (137 mM KCl, 2.7 mM NaCl, 8.1 mM $Na_2HPO_4$, 1.5 mM $KH_2PO_4$ and 4 mM $MgCl_2$; hereinafter referred to as K-PBS buffer). To this suspension was added a test compound appropriately diluted with K-PBS buffer to give a final concentration of 100 µM. The resulting suspension was transferred into a cuvette (width: 0.2 cm, BIO-RAD) and electrical pulses were applied thereto using Shimadzu Cell Fusion Apparatus S-SH-1 (Shimadzu Corporation) under the following conditions: voltage, 3 kV/cm; pulse length, 100 µs; pulse intervals, 1s; number of pulses, 2. After the cuvette was allowed to stand for 10 minutes, the cells were collected and suspended in DMEM-FBS medium. The cell suspension was poured into wells of a 96-well microtiter plate (Nunc, #167008) in an amount of 1 x $10^3$ cells/well, and the same test compound as used above was added to the wells to give a final concentration of 100 µM.

The cells were cultured in the plate at 37°C for 6 days in the presence of 5% $CO_2$. Three hours before the completion of culturing, the medium was removed and the wells were washed once with PBS buffer in an amount of 100 µl/well, followed by addition of 50 µl of DMEM-FBS medium to each well. Then, XTT labelling reagent (Boehringer Mannheim GmbH) prepared by dissolving 1 mg/ml XTT (sodium 3-[1-(phenylaminocarbonyl)-3,4-tetrazolium]-bis(4-methoxy-6-nitro)benzene sulfonic acid hydrate) and 25 µM PMS (N-methyl dibenzopyrazine methylsulfate) in RPMI1640 was added to the wells in an amount of 25 µl/well. After the completion of culturing, the absorbance at 490 nm was measured by using microplate reader Model550 (BIO-RAD) (control wavelength: 655 nm). After the value of the background (without cells) was subtracted, the rate of growth inhibition was calculated according to the following equation using the absorbance measured in the absence of a test compound with application of electrical pulses as a control. The results are shown in Table 5.

Table 5

| Compound | Rate of Growth Inhibition (%) |
|---|---|
| Compound 1 | 16 |
| Compound 4 | 50 |
| Compound 6 | 76 |

Rate of Growth Inhibition = (A-B)/A x 100 (%)

A: Absorbance in the absence of a test compound (A490-A655)
B: Absorbance in the presence of a test compound (A490-A655)
(A490 and A655 refer to the absorbances at 490 nm and 655 nm, respectively)

By the foregoing Test Examples, it was confirmed that the peptides of the present invention are capable of restoring the DNA-binding activity to mutant P53 protein and restoring the P53 protein-dependent transcription activity and have growth inhibiting activity against cancer cells and thus the peptides are useful as therapeutic agents for cancer.

Brief Description of the Drawings

Fig. 1 is an electrophoretic pattern showing the result of gel shift assay using P53His273 protein. The compound number and concentration (µM) of the test compound employed are shown above each lane.
Fig. 2 is an electrophoretic pattern showing the result of gel shift assay using P53Trp248 protein. The compound number and concentration (µM) of the test compound employed are shown above each lane.
Fig. 3 is an electrophoretic pattern showing the result of gel shift assay using A431 cell extract. The compound number and concentration (µM) of the test compound employed are shown above each lane.
Fig. 4 illustrates an outline of the steps for constructing reporter plasmid p53RE1luc2, in which the following abbreviations are used: PSE, core promoter of Sv40 early gene; SPBG, intron derived from rabbit β-globin gene; ABG, poly (A) additional signal derived from rabbit β-globin; ASE, poly (A) additional signal derived from Sv40 early gene; Atk, poly (A) additional signal derived from herpes simplex virus thymidine kinase; G418, G418/kanamycin resistance gene derived from Tn5; Ptk, promoter of herpes simplex virus thymidine kinase; P1, P1 promoter derived from pBR322; Ap, ampicillin resistance gene; p53RE, p53 responsive element; GC, GC box; TATA, TATA box.
Fig. 5 illustrates an outline of the steps for constructing reporter plasmid p53RE2luc2, in which the same abbreviations as in Fig. 4 are used.
Fig. 6 illustrates an outline of the steps for constructing reporter plasmid p53RE4luc2, in which the same abbreviations as in Fig. 4 are used.
Fig. 7 illustrates an outline of the steps for constructing reporter plasmid p53RE8luc2, in which the same abbreviations as in Fig. 4 are used.

Best Modes for Carrying Out the Invention

The physicochemical properties of the compounds shown in Reference Examples and Examples below were determined according to the following methods.
Mass spectrometric analysis was carried out by the FAB-MS method using JEOL JMS-SX102A. Amino acid analysis was carried out by the method of Bidlingmeyer, B.A., et al. [Journal of Chromatography, Vol. 336, p. 93 (1984)]. Hydrolysis was carried out in hydrochloric acid vapor at 110°C for 22 hours. The amino acid compositions of the obtained hydrolyzates were analyzed using Waters Pico Tag amino acid analyzer (Waters Associates).

<u>Reference Example 1</u> Synthesis of Compound 1 (H-Leu-Lys-Ser-Lys-Lys-Gly-Gln-Ser-Thr-Ser-Arg-His-Lys-Lys-Leu-OH)

A carrier resin (Wang resin, 30 mg) combined with 18 μmol of Fmoc-Leu was put in a reactor of an automatic synthesizer (Shimadzu Corporation, PSSM-8) and the following treatments were carried out according to the synthesis program developed by Shimadzu Corporation.

(a) The carrier resin was washed with DMF for 3 minutes and the rinsings were discharged.
(b) To the carrier resin was added 755 μl of a 30% piperidine-DMF solution, and the mixture was stirred for 4 minutes, followed by discharge of said solution. The same treatment was repeated once more.
(c) The carrier resin was washed with DMF for one minute and the rinsings were discharged. The same treatment was repeated 5 times.
Thus, the carrier resin combined with Leu without Fmoc was obtained.
(d) DMF (630 μl) containing 180 μmol of Fmoc-Lys(Boc)-OH, 180 μmol of PyBOP, 180 μmol of HOBt monohydrate and 270 μmol of NMM was stirred for 3 minutes, and the resulting solution was added to the carrier resin. After stirring for 30 minutes, the solution was discharged.
(e) The carrier resin was washed with DMF for one minute. The same treatment was repeated 5 times. Thus, Fmoc-Lys(Boc)-Leu was synthesized on the carrier resin.

Subsequently, washing and deprotection steps (a)-(c) were carried out, and condensation reaction was carried out using Fmoc-Lys(Boc)-OH in step (d), followed by washing step (e) to synthesize Fmoc-Lys(Boc)-Lys(Boc)-Leu on the carrier resin. Then, steps (a)-(e) were repeated to obtain the carrier resin to which a protected peptide was bound. In step (d) in the repeated procedures, Fmoc-His(Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Ser(t-Bu)-OH, Fmoc-Lys(Boc)-OH and Fmoc-Leu-OH were used in turn. After washing and deprotection steps (a)-(c) were carried out, the carrier resin was washed with DMF for one minute. The same treatment was repeated 5 times, and the carrier resin was washed successively with methanol and butyl ether, followed by drying under reduced pressure for 12 hours to obtain the carrier resin to which a side-chain-protected peptide was bound. To the obtained carrier resin was added 600 μl of a mixture of TFA (82.5%), thioanisole (5%), water (5%), ethyl methyl sulfide (3%), 1,2-ethanedithiol (2.5%) and thiophenol (2%), and the resulting mixture was allowed to stand at room temperature for 8 hours to remove the side-chain-protecting groups and to cleave the peptide from the resin. After the resin was separated by filtration, about 10 ml of ether was added to the filtrate, and the deposited precipitate was collected by centrifugation and decantation to obtain 51.8 mg of the crude peptide. This crude product was dissolved in 2 M acetic acid and then purified by HPLC using a reversed-phase column (Shiseido Co., Ltd., CAPCELL PAK C18 30 mm I.D. x 250mm). Elution was carried out with a linear concentration gradient by adding 90% aqueous acetonitrile containing 0.1% TFA to 0.1% aqueous TFA, followed by detection at 220 nm to give a fraction containing the desired peptide. The obtained fraction was lyophilized to give 21.9 mg of Compound 1.

Mass spectrum FAB-MS: 1726.4 ($M + H^+$)
Amino acid analysis: Glx 1.1 (1), Ser 2.7 (3), Gly 1.0 (1), His 1.0 (1), Arg 1.0 (1), Thr 1.0 (1), Leu 1.8 (2), Lys 5.2 (5)

<u>Reference Example 2</u> Synthesis of Compound 2 (H-Lys-Ser-Lys-Lys-Gly-Gln-Ser-Thr-Ser-Arg-His-Lys-Lys-OH)

Condensation was carried out in the same manner as in Reference Example 1 using 30 mg of a carrier resin combined with 18.3 μmol of Fmoc-Lys(Boc) as a starting material, and using Fmoc-Lys (Boc)-OH, Fmoc-His(Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Ser(t-Bu)-OH and Fmoc-Lys(Boc)-OH in turn. The condensation product was washed and dried to obtain the carrier resin to which a side-chain-protected peptide was bound. Removal of the side-chain-protecting groups and cleavage of the peptide from the resin were carried out in the same manner as in Reference Example 1 to obtain 42.7 mg of the crude peptide. The obtained crude peptide was purified by HPLC using a reversed-phase column in the same manner as in Reference Example 1 to give 14.1 mg of Compound 2.

Mass spectrum (FAB-MS) m/z: 1500.2 ($M + H^+$)
Amino acid analysis: Glx 1.1 (1), Ser 2.8 (3), Gly 1.0 (1), His 1.0 (1), Arg 1.0 (1), Thr 1.0 (1), Lys 5.1 (5)

<u>Reference Example 3</u> Synthesis of Compound 3 (H-Lys-Lys-Gly-Gln-Ser-Thr-Ser-Arg-His-Lys-Lys-OH)

Condensation was carried out in the same manner as in Reference Example 1 using 50 mg of a carrier resin

combined with 30.5 μmol of Fmoc-Lys(Boc) as a starting material, and using Fmoc-Lys(Boc)-OH, Fmoc-His (Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH and Fmoc-Lys(Boc)-OH in turn. The condensation product was washed and dried to obtain the carrier resin to which a side-chain-protected peptide was bound. Removal of the side-chain-protecting groups and cleavage of the peptide from the resin were carried out in the same manner as in Reference Example 1 to obtain 54.4 mg of the crude peptide. The obtained crude peptide was purified by HPLC using a reversed-phase column in the same manner as in Reference Example 1 to give 41.4 mg of Compound 3.

Mass spectrum (FAB-MS) m/z: 1284.6 (M + $H^+$)
Amino acid analysis: Glx 1.1 (1), Ser 1.9 (2), Gly 1.1 (1), His 1.0 (1), Arg 0.9 (1), Thr 1.0 (1), Lys 3.8 (4)

Example 1 Synthesis of Compound 4 [H-cyclo(Cys-Leu-Lys-Ser-Lys-Lys-Gly-Gln-Ser-Thr-Ser-Arg-His-Lys-Lys-Leu-Cys)-OH]

The following treatments were carried out using 45.4 mg of a carrier resin combined with 25 μmol of Fmoc-Cys(Trt) as a starting material by the use of a peptide synthesizer (ACT, ACT357).

(a) The carrier resin was washed with 1 ml of DMF for 30 seconds and the rinsings were discharged.
(b) To the carrier resin was added 1 ml of a 25% piperidine-DMF solution, and the mixture was stirred for 2 minutes, followed by discharge of said solution. To the carrier resin was added again 1 ml of a 25% piperidine-DMF solution, and the mixture was stirred for 10 minutes, followed by discharge of said solution. (c) The carrier resin was washed with DMF for 12 seconds, followed by discharge of the rinsings. The same treatment was repeated 7 times. Thus, the carrier resin combined with Cys(Trt) without Fmoc was obtained.
(d) To the carrier resin were added 125 μl of DMF, 500 μl of a NMP solution containing 0.25 M Fmoc-Leu-OH and 0.25 M HOBt monohydrate, 500 μl of a DMF solution containing 0.25 M PyBOP and 125 μl of a DMF solution containing 2.0 M NMM. After stirring for 60 minutes, the solutions were discharged.
(e) The reactor was washed with 500 μl of DMF and then with 1 ml of DMF for 30 seconds, followed by further washing with 500 μl of DMF. Thus, Fmoc-Leu-Cys(Trt) was synthesized on the carrier resin.

Subsequently, washing and deprotection steps (a)-(c) were carried out, and condensation reaction was carried out using a solution containing Fmoc-Lys(Boc)-OH in place of Fmoc-Leu-OH in step (d), followed by washing step (e) to synthesize Fmoc-Lys(Boc)-Leu-Cys(Trt) on the carrier resin. Then, condensation was carried out using, in step (d), Fmoc-Lys(Boc)-OH, Fmoc-His(Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Ser(t-Bu)-OH, Fmoc-Lys (Boc)-OH, Fmoc-Leu-OH and Fmoc-Cys(Trt)-OH in turn. The condensation product was washed and dried to give 145.1 mg of the carrier resin to which a side-chain-protected peptide was bound. Removal of the side-chain-protecting groups and cleavage of the peptide from the resin were carried out in the same manner as in Reference Example 1 to obtain 68.5 mg of the crude peptide. This crude peptide was dissolved in 3 ml of DMF, and 1 ml of dilute aqueous ammonia (pH 9.5) was added thereto. The resulting mixture was allowed to stand at room temperature for 30 minutes and then purified by HPLC using a reversed-phase column (YMC, YMC-Pack ODS-AM 30 mm I.D. x 250 mm) in the same manner as in Reference Example 1 to give 12.7 mg of Compound 4.

Mass spectrum (FAB-MS) m/z: 1930.1 (M + $H^+$)
Amino acid analysis: Glx 1.0 (1), Ser 3.0 (3), Gly 1.1 (1), His 1.1 (1), Arg 1.1 (1), Thr 1.0 (1), Leu 1.7 (2), Lys 5.0 (5), Cys 2.0 (2)

Example 2 Synthesis of Compound 5 [cyclo(Leu-Lys-Ser-Lys-Lys-Gly-Gln-Ser-Thr-Ser-Arg-His-Lys-Lys-Leu)]

To 100 mg of a 2-chlorotritylchloride resin (Shimadzu Corporation) combined with 160 μmol of a Cl group were added a solution prepared by dissolving 80 μmol of Fmoc-Leu-OH in 200 μl of DMF and diluting the resulting solution with 1 ml of DCM, and 10.1 μl of DIEA, followed by stirring at room temperature for 5 minutes. To the mixture were further added 20.3 μl of DIEA and 20.3 μl of DCM, followed by stirring at room temperature for 45 minutes. Then, 53.3 μl of methanol was added to the resulting mixture, followed by stirring for 10 minutes. After being separated by filtration, the resin was washed with DCM, DMF, isopropanol, methanol and butyl ether in turn, and then dried under reduced pressure to obtain Fmoc-Leu-2-chlorotritylchloride resin. To 40 mg of the obtained resin (theoretically combined with 32 μmol of Fmoc-Leu) was added a DMF/DCM (1/1) solution containing 5% piperidine, and the mixture was allowed to stand for 10 minutes, followed by discharge of the solution. Then, condensation was carried out in the same manner as in Reference Example 1 using Fmoc-Lys(Boc)-OH, Fmoc-Lys(Boc)-OH, Fmoc-His(Trt)-OH, Fmoc-Arg(Pmc)-OH,

Fmoc-Ser(Trt)-OH, Fmoc-Thr(Trt)-OH, Fmoc-Ser(Trt)-OH, Fmoc-Gln(Trt)-OH, Fmoc-Gly-OH, Fmoc-Lys(Boc)-OH, Fmoc-Lys(Boc)-OH, Fmoc-Ser(Trt)-OH, Fmoc-Lys(Boc)-OH and Fmoc-Leu-OH in turn. The condensation product was washed and dried to give the carrier resin to which a side-chain-protected peptide was bound. The reaction time for condensation with each amino acid was 40 minutes. Cleavage of the peptide from the resin was carried out in the same manner as in Reference Example 1, except that 0.8 ml of a mixture of acetic acid (20%), TFE (20%) and DCM (60%) was used and the standing time was 2 hours, to give 63.8 mg of the crude peptide with side-chain-protecting groups.

Mass spectrum (FAB-MS) m/z: 3704.9 (M + $H^+$)

To the obtained crude peptide was added 1 ml of a mixed solution of 5% TFA, 5% TFE, 5% triisopropylsilane and 85% DCM, and the resulting mixture was allowed to stand at room temperature for 5 minutes. Then, in the same manner as in Reference Example 1, ether was added to the mixture and the deposited precipitate was collected to give 59.8 mg of the crude peptide in which only the side-chain Trt groups of Ser and Thr were removed.

In 30 ml of DMF was dissolved 30 mg of the obtained crude peptide, and 16.6 mg of PyBOP, 4.9 mg of HOBt monohydrate and 5.3 µl of NMM were added thereto. After the mixture was stirred at 4°C for 18 hours, the solvent was removed under reduced pressure. To the residue was added 1 ml of a mixture of TFA (82.5%), thioanisole (5%), water (5%), ethyl methyl sulfide (3%), 1,2-ethanedithiol (2.5%) and thiophenol (2%), and the mixture was allowed to stand at room temperature for 8 hours. Then, in the same manner as in Reference Example 1, ether was added to the mixture and the deposited precipitate was collected to give 26.6 mg of the crude peptide. The obtained crude peptide was purified by HPLC using a reversed-phase column (Shiseido Co., Ltd., CAPCELL PAK C18 30 mm I.D. x 250 mm) in the same manner as in Reference Example 1 to give 7.7 mg of Compound 5.

Mass spectrum (FAB-MS) m/z: 1708.2 (M + $H^+$)
Amino acid analysis: Glx 1.1 (1), Ser 2.8 (3), Gly 1.2 (1), His 1.0 (1), Arg 1.0 (1), Thr 1.0 (1), Leu 1.8 (2), Lys 5.1 (5)

Example 3 Synthesis of Compound 6 [H-cyclo(Cys-Leu-Lys-Ser-Lys-Lys-Gly-Gln-Ser-Thr-Ser-Arg-His-Lys-Lys-Leu-Cys)-$NH_2$]

Condensation was carried out in the same manner as in Reference Example 1 using 30 mg of a carrier resin (Rink Amide MBHA resin) combined with 16.5 µmol of Fmoc-NH as a starting material, and using Fmoc-Cys(Trt)-OH, Fmoc-Leu-OH, Fmoc-Lys(Boc)-OH, Fmoc-Lys(Boc)-OH, Fmoc-His(Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Ser(t-Bu)-OH, Fmoc-Lys(Boc)-OH, Fmoc-Leu-OH and Fmoc-Cys(Trt)-OH in turn. The condensation product was washed and dried to obtain the carrier resin to which a side-chain-protected peptide was bound. Removal of the side-chain-protecting groups and cleavage of the peptide from the resin were carried out in the same manner as in Reference Example 1 to give 35.8 mg of the crude peptide. The obtained crude peptide was dissolved in 40 ml of water, and dilute aqueous ammonia was added to adjust the solution to pH 7.7, followed by stirring at room temperature for 20 hours. The reaction mixture was purified by HPLC using a reversed-phase column (Shiseido Co., Ltd., CAPCELL PAK C18 30 mm I.D. x 250 mm) in the same manner as in Reference Example 1 to give 3.5 mg of Compound 6.

Mass spectrum (FABMS) m/z: 1931.9 (M + $H^+$)
Amino acid analysis: Glx 0.9 (1), Ser 2.8 (3), Gly 1.0 (1), His 1.2 (1), Arg 1.1 (1), Thr 1.0 (1), Leu 2.1 (2), Lys 4.9 (5), Cys 2.1 (2)

Example 4 Synthesis of Compound 7 [Ac-cyclo(Cys-Leu-Lys-Ser-Lys-Lys-Gly-Gln-Ser-Thr-Ser-Arg-His-Lys-Lys-Leu-Cys)-$NH_2$]

Condensation was carried out in the same manner as in Reference Example 1 using 30 mg of a carrier resin (Rink Amide MBHA resin) combined with 16.5 µmol of Fmoc-NH as a starting material, and using Fmoc-Cys(Trt)-OH, Fmoc-Leu-OH, Fmoc-Lys(Boc)-OH, Fmoc-Lys (Boc)-OH, Fmoc-His(Trt-OH), Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Ser(t-Bu)-OH, Fmoc-Lys(Boc)-OH, Fmoc-Leu-OH and Fmoc-Cys(Trt)-OH in turn. To the condensation product were added 495 µl of DMF and 32.4 µl of acetic anhydride, followed by stirring at room temperature for one hour. Then, the product was washed and dried to obtain the carrier resin to which a side-chain-protected peptide was bound. Removal of the side-chain-protecting groups and cleavage of the peptide from the resin were carried out in the same manner as in Reference Example 1 to give 37.1 mg of the crude peptide. The obtained crude peptide was dissolved in 40 ml of water, and dilute aqueous ammonia was added to adjust the solution to pH 7.7, followed by stirring at room temperature for 20 hours. The resulting mixture was purified by HPLC using a reversed-phase column (Shiseido Co.,

Ltd., CAPCELL PAK C18 30 mm I.D. x 250 mm) in the same manner as in Reference Example 1 to give 4.5 mg of Compound 7.

Mass spectrum (FABMS) m/z: 1974.0 (M + $H^+$)
Amino acid analysis: Glx 0.9 (1), Ser 2.9 (3), Gly 1.0 (1), His 1.2 (1), Arg 1.0 (1), Thr 1.0 (1), Leu 2.0 (2), Lys 4.8 (5), Cys 1.7 (2)

Example 5 Synthesis of Compound 8 [H-cyclo-(Cys-Leu-Lys-Ser-Lys-Lys-Gly-Gln-Ser-Thr-Ser-Arg-His-Lys-Lys-Leu-Cys-Add)-$NH_2$]

Condensation was carried out in the same manner as in Reference Example 1 using 30 mg of a carrier resin (Rink Amide MBHA resin) combined with 16.5 μmol of Fmoc-NH as a starting material, and using Fmoc-Add-OH, Fmoc-Cys(Trt)-OH, Fmoc-Leu-OH, Fmoc-Lys (Boc)-OH, Fmoc-Lys(Boc)-OH, Fmoc-His(Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Ser(t-Bu)-OH, Fmoc-Lys(Boc)-OH, Fmoc-Leu-OH and Fmoc-Cys(Trt)-OH in turn. In step (d), DMF (773 μl) containing 148.5 μmol of each amino acid, 148.5 μmol of HBTU, 148.5 μmol of HOBt monohydrate and 297 μmol of DIEA was stirred for 5 minutes, and the resulting solution was added to the carrier resin, and after stirring for 60 minutes, the solution was discharged. After the completion of condensation reactions, the condensation product was washed and dried to obtain the carrier resin to which a side-chain-protected peptide was bound. Removal of the side-chain-protecting groups and cleavage of the peptide from the resin were carried out in the same manner as in Reference Example 1 to obtain 44.6 mg of the crude peptide. The obtained crude peptide was dissolved in 50 ml of water, and dilute aqueous ammonia was added to adjust the solution to pH 7.5, followed by stirring at room temperature for 18 hours. The reaction mixture was purified by HPLC using a reversed-phase column in the same manner as in Reference Example 1 to give 11.2 mg of Compound 8.

Mass spectrum (FABMS) m/z: 2127.2 (M + $H^+$)
Amino acid analysis: Glx 0.9 (1), Ser 2.9 (3), Gly 1.1 (1), His 1.1 (1), Arg 1.0 (1), Thr 1.0 (1), Leu 2.0 (2), Lys 5.1 (5), Cys 2.2 (2)

Example 6 Synthesis of Compound 11 [H-cyclo-(Cys-Leu-Lys-Ser-Lys-Lys-Gly-Gln-Ser-Thr-Ser-Arg-His-Lys(Ac)-Lys-Leu-Cys-)-$NH_2$]

Condensation was carried out in the same manner as in Reference Example 1 using 50 mg of a carrier resin (Rink Amide MBHA resin) combined with 27.5 μmol of Fmoc-NH as a starting material, and using Fmoc-Cys(Trt)-OH, Fmoc-Leu-OH, Fmoc-Lys(Boc)-OH, Fmoc-Lys(Mtt)-OH, Fmoc-His(Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Ser(t-Bu)-OH, Fmoc-Lys(Boc)-OH, Fmoc-Leu-OH and Boc-Cys(Trt)-OH in turn. In step (d), DMF (1430 μl) containing 275 μmol of each amino acid, 275 μmol of HBTU, 275 μmol of HOBt monohydrate and 550 μmol of DIEA was stirred for 5 minutes, and the resulting solution was added to the carrier resin, and after stirring for 60 minutes, the solution was discharged. A deprotection step after the condensation with Boc-Cys(Trt)-OH was not carried out. After the completion of condensation reactions, the resin was washed with DCM, and 2 ml of a mixture of acetic acid/TFE/DCM (2/2/6) was added thereto. The resulting mixture was allowed to stand at room temperature for 2 hours, followed by discharge of the solution. Then, the product was washed and dried in the same manner as in Reference Example 1 to obtain the carrier resin combined with a side-chain protected peptide in which only the side-chain Mtt group of Lys was removed. To 40% of the obtained resin was added 500 μl of DMF containing 20.8 μl of acetic anhydride and the resulting mixture was stirred at room temperature for 3 hours. After the solution was discharged, the resin was washed and dried. Removal of the side-chain-protecting groups and cleavage of the peptide from the resin were carried out in the same manner as in Reference Example 1 to give 26.4 mg of the crude peptide. The obtained crude peptide was dissolved in 2 M acetic acid and diluted to 35 ml with water, and then dilute aqueous ammonia was added to adjust the solution to pH 7.5, followed by stirring at room temperature for 3 days. The reaction mixture was purified by HPLC using a reversed-phase column in the same manner as in Reference Example 1 to give 2.3 mg of Compound 11.

Mass spectrum (FABMS) m/z: 1971.1 (M + $H^+$)
Amino acid analysis: Glx 0.9 (1), Ser 2.9 (3), Gly 1.1 (1), His 1.3 (1), Arg 1.0 (1), Thr 1.1 (1), Leu 1.8 (2), Lys 4.9 (5), Cys 2.2 (2)

Example 7 Synthesis of Compound 17 [H-cyclo(Cys-Leu-Lys-Ser-Lys-Lys-Gln-Ser-Thr-Ser-Arg-His-Lys-Lys-Leu-Cys)-OH]

Condensation was carried out in the same manner as in Example 1 using 54 mg of a carrier resin combined with 25 μmol of Fmoc-Cys(Trt) as a starting material, and using Fmoc-Leu-OH, Fmoc-Lys(Boc)-OH, Fmoc-Lys(Boc)-OH, Fmoc-His(Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Lys(Boc)-OH, Fmoc-Lys(Boc)-OH, Fmoc-Ser(t-Bu)-OH, Fmoc-Lys(Boc)-OH, Fmoc-Leu-OH and Fmoc-Cys(Trt)-OH in turn. The condensation product was washed and dried to obtain the carrier resin to which a side-chain protected peptide was bound. Removal of the side-chain-protecting groups and cleavage of the peptide from the resin were carried out in the same manner as in Reference Example 1 to give 64.5 mg of the crude peptide. The obtained crude peptide was dissolved in 50 ml of water, and dilute aqueous ammonia was added to adjust the solution to pH 7.6, followed by stirring at room temperature for 2 days. The reaction mixture was purified by HPLC using a reversed-phase column in the same manner as in Reference Example 1 to give 14.1 mg of Compound 17.

Mass spectrum (FABMS) m/z: 1874.0 (M + $H^+$)
Amino acid analysis: Glx 1.0 (1), Ser 2.9 (3), His 1.1 (1), Arg 1.0 (1), Thr 1.0 (1), Leu 2.0 (2), Lys 5.0 (5), Cys 1.5 (2)

Example 8 Synthesis of Compound 18 [cyclo(-$CH_2$CO-Leu-Lys-Ser-Lys-Lys-Gly-Gln-Ser-Thr-Ser-Arg-His-Lys-Lys-Leu-Cys)-$NH_2$]

Condensation was carried out in the same manner as in Reference Example 1 using 50 mg of a carrier resin (Rink Amide MBHA resin) combined with 27.5 μmol of Fmoc-NH as a starting material, and using Fmoc-Cys(Trt)-OH, Fmoc-Leu-OH, Fmoc-Lys(Boc)-OH, Fmoc-Lys(Boc)-OH, Fmoc-His(Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Ser(t-Bu)-OH, Fmoc-Lys(Boc)-OH, Fmoc-Leu-OH and bromoacetic acid in turn. In step (d), DMF (1430 μl) containing 330 μmol of each amino acid, 330 μmol of DIC, 275 μmol of HOBt monohydrate and 275 μmol of DIEA was stirred for 5 minutes, and the resulting solution was added to the carrier resin, and after stirring for 60 minutes, the solution was discharged. A deprotection step after the condensation with bromoacetic acid was not carried out. After the completion of condensation reactions, the resin was washed with methanol and butyl ether and then dried to obtain the resin to which a protected peptide was bound. Removal of the side-chain-protecting groups and cleavage of the peptide from the resin were carried out in the same manner as in Reference Example 1 to obtain 50.7 mg of the crude peptide. The obtained crude peptide was dissolved in 2 M acetic acid and diluted to 50 ml with water, and then dilute aqueous ammonia was added to adjust the solution to pH 7.8, followed by stirring at room temperature for 3 days. The reaction mixture was purified by HPLC using a reversed-phase column in the same manner as in Reference Example 1 to give 4.2 mg of Compound 18.

Mass spectrum (FABMS) m/z: 1869.1 (M + $H^+$)
Amino acid analysis: Glx 0.8 (1), Ser 2.8 (3), Gly 1.1 (1), His 1.3 (1), Arg 1.0 (1), Thr 1.1 (1), Leu 1.9 (2), Lys 5.0 (5), Cys 1.0 (1), Bromoacetic acid was not analyzed.

Example 9 Synthesis of Compound 19

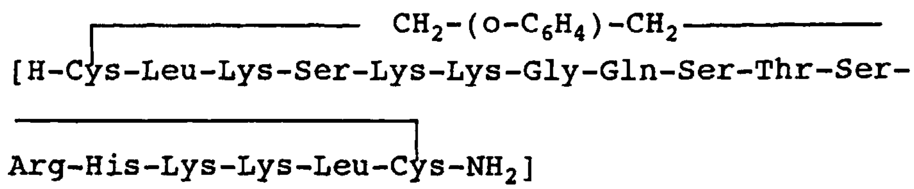

A peptide having the same sequence as Compound 6 was synthesized in the same manner as in Example 3. Condensation with Fmoc-amino acids was carried out using HBTU in place of PyBOP and DIEA in place of NMM. A carrier resin (Rink Amide MBHA resin) (150 mg) combined with 82.5 μmol of Fmoc-NH was used as a starting material, and 197.2 mg of the crude peptide was obtained. The obtained crude peptide wherein the side-chains of 2 Cys residues have free thiol groups was purified by HPLC using a reversed-phase column to give 23.4 mg of the purified peptide.
After 10.2 mg of the purified peptide was dissolved in 100 ml of water containing 0.1% TFA and 10% methanol,

0.5 ml of methanol containing 1.4 mg of o-dibromoxylene was added thereto. The resulting mixture was adjusted to pH 7.5 with dilute aqueous ammonia and then stirred at room temperature for 30 hours. The resulting mixture was purified by HPLC using a reversed-phase column in the same manner as in Reference Example 1 to give 2.2 mg of Compound 19.

Mass spectrum (FAB-MS) m/z: 2034.2 (M + $H^+$)
Amino acid analysis: Glx 0.9 (1), Ser 2.9 (3), Gly 1.1 (1), His 1.1 (1), Arg 1.0 (1), Thr 1.0 (1), Leu 2.0 (2), Lys 4.9 (5), Cys was not analyzed.

Example 10 Synthesis of Compound 20 [H-Leu-Lys-cyclo(Cys-Lys-Lys-Gly-Gln-Ser-Thr-Ser-Arg-His-Lys-Lys-Leu-Cys)-$NH_2$]

Condensation was carried out in the same manner as in Reference Example 1 using 40 mg of a carrier resin (Rink Amide MBHA resin) combined with 22 μmol of Fmoc-NH as a starting material, and using Fmoc-Cys(Trt)-OH, Fmoc-Leu-OH, Fmoc-Lys(Boc)-OH, Fmoc-Lys(Boc)-OH, Fmoc-His(Trt)-OH, Fmoc-Arg(Pmc)-OH, Fmoc-Ser(t-Bu)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Cys(Trt)-OH, Fmoc-Lys(Boc)-OH and Fmoc-Leu-OH in turn. In step (d), DMF (624 μl) containing 220 μmol of each amino acid, 220 μmol of HBTU, 220 μmol of HOBt monohydrate and 440 μmol of DIEA was stirred for 5 minutes, and the resulting solution was added to the carrier resin, and after stirring for 60 minutes, the solution was discharged. After the completion of condensation reactions, the condensation product was washed and dried to obtain the carrier resin to which a side-chain-protected peptide was bound. Removal of the side-chain-protecting groups and cleavage of the peptide from the resin were carried out in the same manner as in Reference Example 1 to give 55.7 mg of the crude peptide. The obtained crude peptide was dissolved in 55.7 ml of water, and dilute aqueous ammonia was added to adjust the solution to pH 7.58, followed by stirring at room temperature for 2 days. The reaction mixture was purified by HPLC using a reversed-phase column in the same manner as in Reference Example 1 to give 10.1 mg of Compound 20.

Mass spectrum (FABMS) m/z: 1843.1 (M + $H^+$)
Amino acid analysis: Glx 0.9 (1), Ser 1.9 (2), Gly 1.0 (1), His 1.2 (1), Arg 1.0 (1), Thr 1.0 (1), Leu 2.0 (2), Lys 5.1 (5), Cys 2.9 (2)

Example 11 Synthesis of Compound 21 [H-cyclo(Cys-Leu-Lys-Ser-Lys-Lys-Gly-Gln-Ser-Thr-Cys)-Arg-His-Lys-Lys-Leu-$NH_2$]

Condensation was carried out in the same manner as in Reference Example 1 using 40 mg of a carrier resin (Rink Amide MBHA resin) combined with 22 μmol of Fmoc-NH as a starting material, and using Fmoc-Leu-OH, Fmoc-Lys(Boc)-OH, Fmoc-Lys(Boc)-OH, Fmoc-His(Trt)-OH, Fmoc-Arg(Pmc)-OH, Fmoc-Cys(Trt)-OH, Fmoc-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Ser(t-Bu)-OH, Fmoc-Lys(Boc)-OH, Fmoc-Leu-OH and Fmoc-Cys(Trt)-OH in turn. Instep (d), DMF (624 μl) containing 220 μmol of each amino acid, 220 μmol of HBTU, 220 μmol of HOBt monohydrate and 440 μmol of DIEA was stirred for 5 minutes, and the resulting solution was added to the carrier resin, and after stirring for 60 minutes, the solution was discharged. After the completion of condensation reactions, the condensation product was washed and dried to obtain the carrier resin to which a side-chain-protected peptide was bound. Removal of the side-chain-protecting groups and cleavage of the peptide from the resin were carried out in the same manner as in Reference Example 1 to give 57.8 mg of the crude peptide. The obtained crude peptide was dissolved in 57.8 ml of water, and dilute aqueous ammonia was added to adjust the solution to pH 7.78, followed by stirring at room temperature for 4 days. The reaction mixture was purified by HPLC using a reversed-phase column in the same manner as in Reference Example 1 to give 2.3 mg of Compound 21.

Mass spectrum (FABMS) m/z: 1843.1 (M + $H^+$)
Amino acid analysis: Glx 0.9 (1), Ser 1.9 (2), Gly 1.0 (1), His 1.1 (1), Arg 1.0 (1), Thr 0.9 (1), Leu 2.0 (2), Lys 5.1 (5), Cys 2.5 (2)

Example 12 Synthesis of Compound 22 [H-Leu-Lys-cyclo(Cys-Lys-Lys-Gly-Gln-Ser-Thr-Cys)-Arg-His-Lys-Lys-Leu-$NH_2$]

Condensation was carried out in the same manner as in Reference Example 1 using 40 mg of a carrier resin (Rink Amide MBHA resin) combined with 22 μmol of Fmoc-NH as a starting material, and using Fmoc-Leu-OH, Fmoc-Lys(Boc)-OH, Fmoc-Lys(Boc)-OH, Fmoc-His(Trt)-OH, Fmoc-Arg(Pmc)-OH, Fmoc-Cys(Trt)-OH, Fmoc-Thr(t-Bu)-OH,

Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Cys(Trt)-OH, Fmoc-Lys(Boc)-OH and Fmoc-Leu-OH in turn. In step (d), DMF (624 μl) containing 220 μmol of each amino acid, 220 μmol of HBTU, 220 μmol of HOBt monohydrate and 440 μmol of DIEA was stirred for 5 minutes, and the resulting solution was added to the carrier resin, and after stirring for 60 minutes, the solution was discharged. After the completion of condensation reactions, the condensation product was washed and dried to obtain the carrier resin to which a side-chain-protected peptide was bound. Removal of the side-chain-protecting groups and cleavage of the peptide from the resin were carried out in the same manner as in Reference Example 1 to give 54.6 mg of the crude peptide. The obtained crude peptide was dissolved in 54.6 ml of water, and dilute aqueous ammonia was added to adjust the solution to pH 7.82, followed by stirring at room temperature for 4 days. The reaction mixture was purified by HPLC using a reversed-phase column in the same manner as in Reference Example 1 to give 7.0 mg of Compound 22.

Mass spectrum (FABMS) m/z: 1755.9 (M + $H^+$)
Amino acid analysis: Glx 0.9 (1), Ser 0.9 (1), Gly 1.0 (1), His 1.1 (1), Arg 1.0 (1), Thr 1.0 (1), Leu 2.0 (2), Lys 5.1 (5), Cys 2.5 (2)

Example 13 Synthesis of Compound 23 [H-cyclo(Cys-Leu-Lys-Ser-Lys-Lys-Gly-Gln-Ser-Thr-Ser-Arg-His-Lys-Lys-Leu-Cys)-Gly-NH-$(CH_2)_3$-$CH_3$]

To 50 mg of a 2-chlorotritylchloride resin (Shimadzu Corporation) combined with 80 μmol of a Cl group were added a solution prepared by dissolving 40 μmol of Fmoc-Gly-OH in 30 μl of DMF and diluting the resulting solution with 720 μl of DCM, and 16 μl of DIEA, followed by stirring at room temperature for 5 minutes. To the mixture were further added 11 μl of DIEA and 11 μl of DCM, followed by stirring at room temperature for one hour. Then, 40 μl of methanol was added to the resulting mixture, followed by stirring for 10 minutes. After being separated by filtration, the resin was washed successively with DCM, DMF, isopropanol, methanol and butyl ether, and then dried under reduced pressure to obtain a Fmoc-Gly-2-chlorotrityl resin. To the obtained resin was added a DMF/DCM (1/1) mixture containing 5% piperidine, and the resulting mixture was allowed to stand for 10 minutes, followed by discharge of the solution. Then, condensation was carried out in the same manner as in Reference Example 1 by the use of an automatic synthesizer using Fmoc-Cys(Trt)-OH, Fmoc-Leu-OH, Fmoc-Lys (Boc)-OH, Fmoc-Lys(Boc)-OH, Fmoc-His(Trt)-OH, Fmoc-Arg(Pmc)-OH, Fmoc-Ser(tBu)-OH, Fmoc-Thr(tBu)-OH, Fmoc-Ser(tBu)-OH, Fmoc-Gln(Trt)-OH, Fmoc-Gly-OH, Fmoc-Lys(Boc)-OH, Fmoc-Lys(Boc)-OH, Fmoc-Ser(tBu)-OH, Fmoc-Lys(Boc)-OH, Fmoc-Leu-OH and Boc-Cys(Trt)-OH in turn. The condensation product was washed and dried to obtain the carrier resin to which a side-chain-protected peptide was bound. The reaction time for condensation with each amino acid was 60 minutes. Cleavage of the peptide from the resin was carried out in the same manner as in Reference Example 1, except that 1.0 ml of a mixture of acetic acid (20%), TFE (20%) and DCM (60%) was used and the standing time was 2 hours, to give 82.9 mg of the crude peptide having the side-chain- and N-terminal-protecting groups. The obtained crude peptide (41.5 mg) was added to 1.5 ml of DMF containing 13 mg of PyBOP, 3.2 mg of HOBt monohydrate, 2.6 μl of NMM and 2.5 μl of n-butylamine, and the resulting mixture was allowed to stand at room temperature for 60 hours, followed by evaporation of DMF under reduced pressure to obtain the crude peptide-n-butylamide. Removal of the side-chain- and N-terminal-protecting groups was carried out using 0.8 ml of a mixed solvent of TFA (82.5%), water (5%), thioanisole (5%), ethyl methyl sulfide (3%), 1,2-ethanedithiol (2.5%) and thiophenol (2%), and after the resulting mixture was allowed to stand at room temperature for 8 hours, in the same manner as in Reference Example 1, ether was added and the deposited precipitate was collected to obtain 31.8 mg of the crude peptide. The obtained crude peptide was purified by HPLC using a reversed-phase column in the same manner as in Reference Example 1 to give 7.5 mg of the purified peptide wherein the side-chains of two Cys residues have free thiol groups. The obtained purified peptide was dissolved in 0.75 ml of 2 M acetic acid and diluted with 6.75 ml of water, and then dilute aqueous ammonia was added to adjust the solution to pH 7.5, followed by stirring at room temperature for 48 hours. The reaction mixture was purified by HPLC using a reversed-phase column in the same manner as in Reference Example 1 to give 5.0 mg of Compound 23.

Mass spectrum (FAB-MS) m/z: 2042.8 (M + $H^+$)
Amino acid analysis: Glx 1.0 (1), Ser 2.8 (3), Gly 2.1 (2), His 1.2 (1), Arg 1.0 (1), Thr 1.0 (1), Leu 2.0 (2), Lys 5.0 (5), Cys 2.5 (2)

Example 14 Synthesis of Compound 24 [H-Leu-Lys-cyclo(Asp-Lys-Lys-Gly-Gln-Ser-Thr-Ser-Arg-His-Lys)-Lys-Leu-$NH_2$]

Condensation was carried out in the same manner as in Reference Example 1 using 100 mg of a carrier resin (Rink Amide TGR resin, NovaBiochem) combined with 20 μmol of Fmoc-NH as a starting material, and using Fmoc-Leu-OH, Fmoc-Lys(Boc)-OH, Fmoc-Lys(Aloc)-OH, Fmoc-His(Trt)-OH, Fmoc-Arg(Pmc)-OH, Fmoc-Ser(t-Bu)-OH,

FmoC-Thr(t-Bu)-OH, Fmoc-Ser(t-Bu)-OH, Fmoc-Gln(Trt)-OH, Fmoc-Gly-OH, Fmoc-Lys(Boc)-OH, Fmoc-Lys(Boc)-OH, Fmoc-Asp(OAl)-OH, Fmoc-Lys(Boc)-OH and Boc-Leu-OH in turn. The condensation product was washed and dried to obtain the carrier resin to which a side-chain-protected peptide was bound. To the obtained resin was added a solution prepared by dissolving 173 mg of $PdP(Ph_3)_4$ in a mixed solvent of $CHCl_3$/NMM/acetic acid (92.5/2.5/5), and the resulting mixture was allowed to stand at room temperature for 2 hours. After the solution was discharged, the resin was washed with a DMF solution containing 0.5% DIEA, a DMF solution containing 0.5% sodium diethyl dithiocarbamate, methanol and butyl ether 3 times each to selectively remove the side-chain-protecting allyl groups (Aloc, Al). The resin was then suspended in 1 ml of DMF, and 32 mg of HATU and 24 μl of DIEA were added thereto, followed by stirring at room temperature for 24 hours. After discharge of the solution, the resin was washed and dried in the same manner as in Reference Example 1 and the peptide was cleaved. To the obtained mixture was added ether and the deposited precipitate was collected to obtain 60.4 mg of the crude peptide. The obtained crude peptide was purified by HPLC using a reversed-phase column in the same manner as in Reference Example 1 to give 1.8 mg of Compound 24.

Mass spectrum (FAB-MS) m/z: 1735.9 ($M + H^+$)
Amino acid analysis: Asx 0.6 (1), Glx 1.0 (1), Ser 2.1 (2), Gly 1.2 (1), His 1.2 (1), Arg 1.1 (1), Thr 1.1 (1), Leu 2.0 (2), Lys 4.8 (5)

Industrial Applicability

The present invention provides peptides having a cyclic structure and pharmaceutically acceptable salts thereof. Said peptides directly act on mutant P53 protein to enhance the DNA-binding activity and to restore and enhance the transcription activity, and thereby exhibit antitumor activity.

# Sequence Listing

(1)GENERAL INFORMATION
APPLICANT
KYOWA HAKKO KOGYO CO. LTD.
TITLE OF INVENTION
Peptides having a cyclic structure and restoring the activities of P53 protein to mutant P53 protein
FILE REFERENCE
1061
CURRENT FILING DATE
1998.5.15
PRIOR APPLICATION NUMBER
JP 126113/97
PRIOR APPLICATION FILING DATE
1997.5.15
NUMBER OF SEQUENCES
32
(2)INFORMATION FOR SEQUENCES
INFORMATION FOR SEQ ID NO:1:
LENGTH: 15
TYPE: amino acid
STRANDEDNES: single
TOPOLOGY: linear
MOLECULE TYPE: peptide
SEQUENCE DESCRIPTION:

```
Leu Lys Ser Lys Lys Gly Gln Ser Thr Ser Arg His Lys Lys Leu
  1               5                  10                  15
```

INFORMATION FOR SEQ ID NO:2:
LENGTH: 13
TYPE: amino acid
STRANDEDNES: single
TOPOLOGY: linear
MOLECULE TYPE: peptide
SEQUENCE DESCRIPTION:

```
Lys Ser Lys Lys Gly Gln Ser Thr Ser Arg His Lys Lys
  1               5                  10
```

INFORMATION FOR SEQ ID NO:3:
LENGTH: 11
TYPE: amino acid
STRANDEDNES: single
TOPOLOGY: linear
MOLECULE TYPE: peptide
SEQUENCE DESCRIPTION:

```
Lys Lys Gly Gln Ser Thr Ser Arg His Lys Lys
  1               5                  10
```

INFORMATION FOR SEQ ID NO:4:
LENGTH: 17
TYPE: amino acid
STRANDEDNES: single
TOPOLOGY: linear
MOLECULE TYPE: peptide
FEATURE:
NAME/KEY: disulfide-bonds
LOCATION: 1 and 17
SEQUENCE DESCRIPTION:

Cys Leu Lys Ser Lys Lys Gly Gln Ser Thr Ser Arg His Lys Lys Leu
1 5 10 15
Cys

INFORMATION FOR SEQ ID NO:5:
LENGTH: 15
TYPE: amino acid
STRANDEDNES: single
TOPOLOGY: circular
MOLECULE TYPE: peptide
SEQUENCE DESCRIPTION:
Leu Lys Ser Lys Lys Gly Gln Ser Thr Ser Arg His Lys Lys Leu
1 5 10 15
INFORMATION FOR SEQ ID NO:6:
LENGTH: 17
TYPE: amino acid
STRANDEDNES: single
TOPOLOGY: linear
MOLECULE TYPE: peptide
FEATURE:
NAME/KEY: disulfide-bonds
LOCATION: 1 and 17
NAME/KEY: modified site
LOCATION: 17
OTHER INFORMATION: Xaa represents L-Cysteine amide
SEQUENCE DESCRIPTION:
Cys Leu Lys Ser Lys Lys Gly Gln Ser Thr Ser Arg His Lys Lys Leu
1 5 10 15
Xaa

INFORMATION FOR SEQ ID NO:7:
LENGTH: 17
TYPE: amino acid
STRANDEDNES: single
TOPOLOGY: linear
MOLECULE TYPE: peptide
FEATURE:
NAME/KEY: disulfide-bonds
LOCATION: 1 and 17
NAME/KEY: modified site
LOCATION: 1
OTHER INFORMATION: Xaa represents N-Acetyl-L-cysteine
NAME/KEY: modified site
LOCATION: 17
OTHER INFORMATION: Xaa represents L-Cysteine amide
SEQUENCE DESCRIPTION:
Xaa Leu Lys Ser Lys Lys Gly Gln Ser Thr Ser Arg His Lys Lys Leu
1 5 10 15
Xaa

INFORMATION FOR SEQ ID NO:8:
LENGTH: 32
TYPE: nucleic acid
STRANDEDNES: single
TOPOLOGY: linear
MOLECULE TYPE: other nucleic acid synthetic DNA
FEATURE: A determined method of the sequence : E
SEQUENCE DESCRIPTION:

CTAGACAGCC AGACTGCCTT CCGGGTCACT GC 32

INFORMATION FOR SEQ ID NO:9:
LENGTH: 32
TYPE: nucleic acid
STRANDEDNES: single
TOPOLOGY: linear
MOLECULE TYPE: other nucleic acid synthetic DNA
FEATURE: A determined method of the sequence : E
SEQUENCE DESCRIPTION:
CATGGCAGTG ACCCGGAAGG CAGTCTGGCT GT 32

INFORMATION FOR SEQ ID NO:10:
LENGTH: 26
TYPE: nucleic acid
STRANDEDNES: single
TOPOLOGY: linear
MOLECULE TYPE: other nucleic acid synthetic DNA
FEATURE: A determined method of the sequence : E
SEQUENCE DESCRIPTION:
TCGAGAGACA TGCCTAGACA TGCCTG 26

INFORMATION FOR SEQ ID NO:11:
LENGTH: 26
TYPE: nucleic acid
STRANDEDNES: single
TOPOLOGY: linear
MOLECULE TYPE: other nucleic acid synthetic DNA
FEATURE: A determined method of the sequence : E
SEQUENCE DESCRIPTION:
TCGACAGGCA TGTCTAGGCA TGTCTC 26

INFORMATION FOR SEQ ID NO:12:
LENGTH: 22
TYPE: nucleic acid
STRANDEDNES: single
TOPOLOGY: linear
MOLECULE TYPE: other nucleic acid synthetic DNA
FEATURE: A determined method of the sequence : E
SEQUENCE DESCRIPTION:
TCGAGCCCGG GGGTACCGCA TG 22

INFORMATION FOR SEQ ID NO:13:
LENGTH: 14
TYPE: nucleic acid
STRANDEDNES: single
TOPOLOGY: linear
MOLECULE TYPE: other nucleic acid synthetic DNA
FEATURE: A determined method of the sequence : E
SEQUENCE DESCRIPTION:
CGGTACCCCC GGGC 14

INFORMATION FOR SEQ ID NO:14:
LENGTH: 32
TYPE: nucleic acid
STRANDEDNES: single
TOPOLOGY: linear
MOLECULE TYPE: other nucleic acid synthetic DNA

FEATURE: A determined method of the sequence : E
SEQUENCE DESCRIPTION:
TCGAGGGACT TGCCTGGACT TGCCTGTCGA CG 32

INFORMATION FOR SEQ ID NO:15:
LENGTH: 32
TYPE: nucleic acid
STRANDEDNES: single
TOPOLOGY: linear
MOLECULE TYPE: other nucleic acid synthetic DNA
FEATURE: A determined method of the sequence : E
SEQUENCE DESCRIPTION:
GTACCGTCGA CAGGCAAGTC CAGGCAAGTC CC 32

INFORMATION FOR SEQ ID NO:16:
LENGTH: 18
TYPE: amino acid
STRANDEDNES: single
TOPOLOGY: linear
MOLECULE TYPE: peptide
FEATURE:
NAME/KEY: disulfide-bonds
LOCATION: 1 and 17
NAME/KEY: modified site
LOCATION: 18
OTHER INFORMATION: Xaa represents 12-Dodecanamide
SEQUENCE DESCRIPTION:
Cys Leu Lys Ser Lys Lys Gly Gln Ser Thr Ser Arg His Lys Lys Leu
1 5 10 15
Cys Xaa

INFORMATION FOR SEQ ID NO:17:
LENGTH: 17
TYPE: amino acid
STRANDEDNES: single
TOPOLOGY: linear
MOLECULE TYPE: peptide
NAME/KEY: disulfide-bonds
LOCATION: 1 and 17
NAME/KEY: modified site
LOCATION: 17
OTHER INFORMATION: Xaa represents N-Dodecyl-L-cysteine amide
SEQUENCE DESCRIPTION:
Cys Leu Lys Ser Lys Lys Gly Gln Ser Thr Ser Arg His Lys Lys Leu
1 5 10 15
Xaa

INFORMATION FOR SEQ ID NO:18:
LENGTH: 17
TYPE: amino acid
STRANDEDNES: single
TOPOLOGY: linear
MOLECULE TYPE: peptide
NAME/KEY: disulfide-bonds
LOCATION: 1 and 17
NAME/KEY: modified site
LOCATION: 17
OTHER INFORMATION: Xaa represents N-Octadodecyl-L-cysteine amide

SEQUENCE DESCRIPTION:
Cys Leu Lys Ser Lys Lys Gly Gln Ser Thr Ser Arg His Lys Lys Leu
1 5 10 15
Xaa

INFORMATION FOR SEQ ID NO:19:
LENGTH: 17
TYPE: amino acid
STRANDEDNES: single
TOPOLOGY: linear
MOLECULE TYPE: peptide
NAME/KEY: disulfide-bonds
LOCATION: 1 and 17
NAME/KEY: modified site
LOCATION: 14
OTHER INFORMATION: Xaa represents $N^{\varepsilon}$-Acetyl-L-lysine
SEQUENCE DESCRIPTION:
INFORMATION FOR SEQ ID NO:19:
Cys Leu Lys Ser Lys Lys Gly Gln Ser Thr Ser Arg His Xaa Lys Leu
1 5 10 15
Cys

INFORMATION FOR SEQ ID NO:20:
LENGTH: 17
TYPE: amino acid
STRANDEDNES: single
TOPOLOGY: linear
MOLECULE TYPE: peptide
NAME/KEY: $-SCH_2S-$ bond
LOCATION: 1 and 17
NAME/KEY: modified site
LOCATION: 1
OTHER INFORMATION: Xaa represents L-Cysteine
LOCATION: 17
OTHER INFORMATION: Xaa represents L-Cysteine amide
SEQUENCE DESCRIPTION:
Xaa Leu Lys Ser Lys Lys Gly Gln Ser Thr Ser Arg His Lys Lys Leu
1 5 10 15
Xaa

INFORMATION FOR SEQ ID NO:21:
LENGTH: 16
TYPE: amino acid
STRANDEDNES: single
TOPOLOGY: both
MOLECULE TYPE: peptide
NAME/KEY: cross-links
LOCATION: 1 and 16
OTHER INFORMATION: $-NH_2(\varepsilon)$in Lys and -COOH in Leu form an amide bond.
SEQUENCE DESCRIPTION:
Lys Leu Lys Ser Lys Lys Gly Gln Ser Thr Ser Arg His Lys Lys Leu
1 5 10 15
INFORMATION FOR SEQ ID NO:22:
LENGTH: 16
TYPE: amino acid
STRANDEDNES: single
TOPOLOGY: linear
MOLECULE TYPE: peptide

NAME/KEY: disulfide-bonds
LOCATION: 1 and 8
NAME/KEY: modified site
LOCATION: 16
OTHER INFORMATION: Xaa represents L-Leucine amide
SEQUENCE DESCRIPTION:
Cys Leu Lys Ser Lys Lys Gly Cys Ser Thr Ser Arg His Lys Lys Xaa
1 5 10 15

INFORMATION FOR SEQ ID NO:23:
LENGTH: 16
TYPE: amino acid
STRANDEDNES: single
TOPOLOGY: both
MOLECULE TYPE: peptide
NAME/KEY: cross-links
LOCATION: 1 and 8
OTHER INFORMATION: $-NH_2(\varepsilon)$in Lys and -COOH in Asp form an amide bond.
NAME/KEY: modified site
LOCATION: 16
OTHER INFORMATION: Xaa represents L-Leucine amide
SEQUENCE DESCRIPTION:
Lys Leu Lys Ser Lys Lys Gly Asp Ser Thr Ser Arg His Lys Lys Xaa
1 5 10 15
INFORMATION FOR SEQ ID NO:24:
LENGTH: 16
TYPE: amino acid
STRANDEDNES: single
TOPOLOGY: both
MOLECULE TYPE: peptide
NAME/KEY: cross-links
LOCATION: 7 and 13
NAME/KEY: modified site
LOCATION: 15
OTHER INFORMATION: Xaa represents L-Leucine amide
SEQUENCE DESCRIPTION:
Leu Lys Ser Lys Lys Gly Asp Ser Thr Ser Arg His Lys Lys Xaa
1 5 10 15
INFORMATION FOR SEQ ID NO:25:
LENGTH: 16
TYPE: amino acid
STRANDEDNES: single
TOPOLOGY: linear
MOLECULE TYPE: peptide
NAME/KEY: disulfide-bonds
LOCATION: 1 and 16
SEQUENCE DESCRIPTION:
Cys Leu Lys Ser Lys Lys Gln Ser Thr Ser Arg His Lys Lys Leu Cys
1 5 10 15
INFORMATION FOR SEQ ID NO:26:
LENGTH: 16
TYPE: amino acid
STRANDEDNES: single
TOPOLOGY: linear
MOLECULE TYPE: peptide
NAME/KEY: $-SCH_2-$ bond
LOCATION: 1 and 16
NAME/KEY: modified site

LOCATION: 1
OTHER INFORMATION: Xaa represents N-Methylenecarbonyl-L-Leucine whose methylene bonds to S in L-Cysteine amide.
NAME/KEY: modified site
LOCATION: 16
OTHER INFORMATION: Xaa represents L-Cysteine amide whose S bonds to methylene in N-Methylenecarbonyl-L-leucine.
SEQUENCE DESCRIPTION:
Xaa Lys Ser Lys Lys Gly Gln Ser Thr Ser Arg His Lys Lys Leu Xaa
1 5 10 15
INFORMATION FOR SEQ ID NO:27:
LENGTH: 17
TYPE: amino acid
STRANDEDNES: single
TOPOLOGY: linear
MOLECULE TYPE: peptide
NAME/KEY: -S-(o-xylylene)-S- bond
LOCATION: 1 and 17
NAME/KEY: modified site
LOCATION: 1
OTHER INFORMATION: Xaa represents L-Cysteine.
NAME/KEY: modified site
LOCATION: 17
OTHER INFORMATION: Xaa represents L-Cysteine amide.
SEQUENCE DESCRIPTION:
Xaa Leu Lys Ser Lys Lys Gly Gln Ser Thr Ser Arg His Lys Lys Leu
1 5 10 15
Xaa

INFORMATION FOR SEQ ID NO:28:
LENGTH: 16
TYPE: amino acid
STRANDEDNES: single
TOPOLOGY: linear
MOLECULE TYPE: peptide
NAME/KEY: disulfide-bonds
LOCATION: 3 and 16
NAME/KEY: modified site
LOCATION: 16
OTHER INFORMATION: Xaa represents L-Cysteine amide.
SEQUENCE DESCRIPTION
Leu Lys Cys Lys Lys Gly Gln Ser Thr Ser Arg His Lys Lys Leu Xaa
1 5 10 15
INFORMATION FOR SEQ ID NO:29:
LENGTH: 16
TYPE: amino acid
STRANDEDNES: single
TOPOLOGY: linear
MOLECULE TYPE: peptide
NAME/KEY: disulfide-bonds
LOCATION: 1 and 11
NAME/KEY: modified site
LOCATION: 16
OTHER INFORMATION: Xaa represents L-Leucine amide.
SEQUENCE DESCRIPTION:
Cys Leu Lys Ser Lys Lys Gly Gln Ser Thr Cys Arg His Lys Lys Xaa
1 5 10 15

INFORMATION FOR SEQ ID NO:30:
LENGTH: 15
TYPE: amino acid
STRANDEDNES: single
TOPOLOGY: linear
MOLECULE TYPE: peptide
NAME/KEY: disulfide-bonds
LOCATION: 3 and 10
NAME/KEY: modified site
LOCATION: 15
OTHER INFORMATION: Xaa represents L-Leucine amide.
SEQUENCE DESCRIPTION:

```
Leu Lys Cys Lys Lys Gly Gln Ser Thr Cys Arg His Lys Lys Xaa
 1               5                  10                  15
```

INFORMATION FOR SEQ ID NO:31:
LENGTH: 18
TYPE: amino acid
STRANDEDNES: single
TOPOLOGY: linear
MOLECULE TYPE: peptide
NAME/KEY: disulfide-bonds
LOCATION: 1 and 17
NAME/KEY: modified site
LOCATION: 18
OTHER INFORMATION: Xaa represents L-Glycine n-butyl amide.
SEQUENCE DESCRIPTION:

```
Cys Leu Lys Ser Lys Lys Gly Gln Ser Thr Ser Arg His Lys Lys Leu
 1               5                  10                  15
Cys Xaa
```

INFORMATION FOR SEQ ID NO:32:
LENGTH: 15
TYPE: amino acid
STRANDEDNES: single
TOPOLOGY: both
MOLECULE TYPE: peptide
NAME/KEY: cross-links
LOCATION: 3 and 13
OTHER INFORMATION: $-NH_2(\varepsilon)$ in Lys and $-COOH(\beta)$ in Asp form an amide bond.
NAME/KEY: modified site
LOCATION: 15
OTHER INFORMATION: Xaa represents L-Leucine amide.
SEQUENCE DESCRIPTION:

```
Leu Lys Asp Lys Lys Gly Gln Ser Thr Ser Arg His Lys Lys Xaa
 1               5                  10                  15
```

## Claims

1. A peptide having a cyclic structure and having an activity to restore the DNA-binding activity or the P53 protein-dependent transcription activity to mutant P53 protein, or a pharmaceutically acceptable salt thereof, said peptide being represented by general formula (I):

$$R^1(X^1)^{n1}(X^2)^{n2}(X^3)^{n3}(X^4)^{n4}(X^5)^{n5}(X^6)^{n6}(X^7)^{n7}(X^8)^{n8}(X^9)^{n9}(X^{10})^{n10}(X^{11})^{n11}(X^{12})^{n12}(X^{13})^{n13}(X^{14})^{n14}(X^{15})^{n15}(X^{16})^{n16}(X^{17})^{n17}R^2 \quad (I)$$

{wherein any of $X^1$ to $X^{17}$ and n1 to n17 may be denoted by $X^i$ and ni, respectively (i stands for an integer of 1 to 17); $X^i$ represents an amino acid residue or an organic acid residue as defined below; ni represents 0 or 1; $(X^i)^{ni}$ represents $X^i$ when ni is 1, and represents a bond when ni is 0; 7 to 17 different $X^i$s (ni=1) are selected, arranged in order of increasing number i, and bonded to one another, with $R^1$ bonded to the N-terminus and $R^2$ bonded to the C-terminus, to represent one sequence, in which a functional group in residue $X^p$ (p is an integer of 1 to 11) is selected from the group of $X^1$ to $X^{11}$ and a functional group in residue $X^q$ (q is an integer of 8 to 17, provided that q is larger than p) is selected from the group of $X^8$ to $X^{17}$ form a cyclic structure; $R^1$ represents substituted or unsubstituted alkanoyl, substituted or unsubstituted alkoxycarbonyl, substituted or unsubstituted aralkyloxycarbonyl, substituted or unsubstituted aryloxycarbonyl, substituted or unsubstituted aroyl, 9-fluorenylmethoxycarbonyl, or hydrogen; $X^1$ represents a residue of 2-mercaptobenzoic acid, 3-mercaptopropionic acid, 4-mercaptobutanoic acid, mercaptoacetic acid, adipic acid, suberic acid, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, serine, threonine, homoserine, α-methylserine, 3-hydroxyproline or 4-hydroxyproline; $X^2$ represents a residue of leucine, isoleucine, valine, alanine, norvaline, norleucine, 2-aminobutanoic acid, homoleucine, β-alanine, α-aminoisobutanoic acid, β-cyclopropylalanine, β-chloroalanine, 1-aminocyclopentane-1-carboxylic acid, 1-amino-1-cyclohexanecarboxylic acid, 2-amino-1-cyclopentanecarboxylic acid, t-butylglycine, diethylglycine, t-butylalanine, O-methylserine, cyclohexylglycine, cyclohexylalanine or glycine; $X^3$ represents a residue of lysine, arginine, ornithine, 2,4-diaminobutanoic acid, 2,3-diaminopropionic acid, p-aminophenylalanine or glycine; $X^4$ represents a residue of serine, threonine, homoserine, α-methylserine, 3-hydroxyproline, 4-hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, glycine, 2-mercaptobenzoic acid, 3-mercaptopropionic acid, 4-mercaptobutanoic acid, mercaptoacetic acid, adipic acid or suberic acid; $X^5$ represents a residue of lysine, arginine, ornithine, 2,4-diaminobutanoic acid, 2,3-diaminopropionic acid, p-aminophenylalanine or glycine; $X^6$ represents a residue of lysine, arginine, ornithine, 2,4-diaminobutanoic acid, 2,3-diaminopropionic acid, p-aminophenylalanine or glycine; $X^7$ represents a residue of alanine, β-alanine, 2-aminobenzoic acid, 3-aminobenzoic acid, 4-aminobenzoic acid, 3-aminomethylbenzoic acid, proline, 3-hydroxyproline, 4-hydroxyproline, L-1,2,3,4-tetrahydroisoquinoline-7-carboxylic acid, cysteine, homocysteine, penicillamine, 2,3-diaminopropionic acid, 2,4-diaminobutanoic acid, ornithine, lysine, p-aminophenylalanine, aspartic acid, glutamic acid, isoaspartic acid, isoglutamic acid, 2-aminoadipic acid, 2-aminosuberic acid or glycine; $X^8$ represents a residue of glutamine, asparagine, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, serine, threonine, homoserine, α-methylserine, 3-hydroxyproline, 4-hydroxyproline, glycine, 2-mercaptobenzoic acid, 3-mercaptopropionic acid, 4-mercaptobutanoic acid, mercaptoacetic acid, adipic acid or suberic acid; $X^9$ represents a residue of serine, threonine, homoserine, α-methylserine, 3-hydroxyproline, 4-hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, glycine, 2-mercaptobenzoic acid, 3-mercaptopropionic acid, 4-mercaptobutanoic acid, mercaptoacetic acid, adipic acid or suberic acid; $X^{10}$ represents a residue of serine, threonine, homoserine, α-methylserine, hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, glycine, 2-mercaptobenzoic acid, 3-mercaptopropionic acid, 4-mercaptobutanoic acid, mercaptoacetic acid, adipic acid or suberic acid; $X^{11}$ represents a residue of serine, threonine, homoserine, α-methylserine, hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine, glycine, 2-mercaptobenzoic acid, 3-mercaptopropionic acid, 4-mercaptobutanoic acid, mercaptoacetic acid, adipic acid or suberic acid; $X^{12}$ represents a residue of lysine, arginine, ornithine, 2,4-diaminobutanoic acid, 2,3-diaminopropionic acid, p-aminophenylalanine or glycine; $X^{13}$ represents a residue of histidine, alanine, 4-thiazolylalanine, 2-thienylalanine, 2-pyridylalanine, 3-pyridylalanine, 4-pyridylalanine, (3-N-methyl)piperidylalanine, 3- (2-quinoyl)alanine, serine, threonine, homoserine, α-methylserine, 3-hydroxyproline, 4-hydroxyproline, cysteine, homocysteine, penicillamine, aspartic acid, glutamic acid, homoglutamic acid, isoaspartic acid, isoglutamic acid, 2-aminoadipic acid, 2-aminosuberic acid, ornithine, lysine, 2,4-diaminobutanoic acid, 2,3-diaminopropionic acid, p-aminophenylalanine or glycine; $X^{14}$ represents a residue of lysine, arginine, ornithine, 2,4-diaminobutanoic acid, 2,3-diaminopropionic acid, p-aminophenylalanine, serine, threonine, homoserine, α-methylserine, 3-hydroxyproline, 4-hydroxyproline, cysteine, homocysteine, penicillamine, aspartic acid, glutamic acid, homoglutamic acid, isoaspartic acid, isoglutamic acid, 2-aminoadipic acid, 2-aminosuberic acid or glycine, and an amino group or guanidino group in the side chain of $X^{14}$ may be modified with $R^3$

($R^3$ has the same significance as $R^1$); $X^{15}$ represents lysine, arginine, ornithine, 2,4-diaminobutanoic acid, 2,3-diaminopropionic acid, p-aminophenylalanine or glycine; $X^{16}$ represents a residue of leucine, alanine, 4-thiazolylalanine, 2-thienylalanine, isoleucine, norleucine, homoleucine, valine, norvaline, β-alanine, α-aminoisobutanoic acid, 2-aminobutanoic acid, β-cyclopropylalanine, β-chloroalanine, 1-aminocyclopentane-1-carboxylic acid, 1-amino-1-cyclohexanecarboxylic acid, 2-amino-1-cyclopentanecarboxylic acid, t-butylglycine, diethylglycine, t-butylalanine, O-methylserine, cyclohexylglycine, cyclohexylalanine or glycine; $X^{17}$ represents a residue of 2-mercaptoaniline, cysteamine, homocysteamine, cysteine, homocysteine, penicillamine, ornithine, lysine, 2,3-diaminopropionic acid, 2,4-diaminobutanoic acid, p-aminophenylalanine, glutamic acid, aspartic acid, homoglutamic acid, isoaspartic acid, isoglutamic acid, 2-aminoadipic acid or 2-aminosuberic acid; $R^2$ represents substituted or unsubstituted alkoxy, substituted or unsubstituted aralkyloxy, amino, substituted or unsubstituted alkylamino, substituted or unsubstituted dialkylamino, substituted or unsubstituted aralkylamino, substituted or unsubstituted arylamino, or hydroxy; and one to several residues which are the same or different and arbitrarily selected from the group consisting of organic acid residues, amino acid residues and a 12-aminododecanoic acid residue mentioned in the above $X^i$ representations may be deleted, substituted or added at arbitrary positions in the sequence}.

**2.** A peptide having a cyclic structure or a pharmaceutically acceptable salt thereof according to claim 1, wherein said cyclic structure is formed by a S-S, $S-CH_2-S$, $S-CH_2-C_6H_4-CH_2-S$, $S-CH_2-CO$, CO-NH, NH-CO, O-CO or CO-O bond between $X^p$ and $X^q$.

**3.** A peptide having a cyclic structure or a pharmaceutically acceptable salt thereof according to claim 2, wherein $X^p$ (np=1) is an N-terminal residue and $x^q$ (nq=1) is a C-terminal residue.

**4.** A peptide having a cyclic structure or a pharmaceutically acceptable salt thereof according to claim 2, wherein $X^p$ (np=1) is not an N-terminal residue and $X^q$ (nq=1) is not a C-terminal residue.

**5.** A peptide having a cyclic structure or a pharmaceutically acceptable salt thereof according to claim 2, wherein $X^p$ (np=1) is not an N-terminal residue and $x^q$ (nq=1) is a C-terminal residue.

**6.** A peptide having a cyclic structure or a pharmaceutically acceptable salt thereof according to claim 2, wherein $X^p$ (np=1) is an N-terminal residue and $X^q$ (nq=1) is not a C-terminal residue.

**7.** A peptide having a cyclic structure or a pharmaceutically acceptable salt thereof according to claim 3, wherein $X^p$ (np=1) is $X^1$ and $X^q$ (nq=1) is $X^{17}$.

**8.** A peptide having a cyclic structure or a pharmaceutically acceptable salt thereof according to claim 6, wherein $X^p$ (np=1) is $X^1$ and $X^q$ (nq=1) is $X^{17}$.

**9.** A peptide having a cyclic structure or a pharmaceutically acceptable salt thereof according to claim 3, wherein $X^p$ (np=1) is $X^1$ and $X^q$ (nq=1) is $X^{16}$.

**10.** A peptide having a cyclic structure or a pharmaceutically acceptable salt thereof according to claim 6, wherein $X^p$ (np=1) is an N-terminal residue and $X^q$ (nq=1) is $X^8$.

**11.** A peptide having a cyclic structure or a pharmaceutically acceptable salt thereof according to claim 4, wherein $X^p$ (np=1) is $X^8$ and $X^q$ (nq=1) is $X^{14}$.

**12.** A peptide having a cyclic structure or a pharmaceutically acceptable salt thereof according to claim 5, wherein $X^p$ (np=1) is $X^3$ and $X^q$ (nq=1) is a C-terminal residue.

**13.** A peptide having a cyclic structure or a pharmaceutically acceptable salt thereof according to claim 4, wherein $X^p$ (np=1) is $X^3$ and $X^q$ (nq=1) is not a C-terminal residue.

**14.** A peptide having a cyclic structure or a pharmaceutically acceptable salt thereof according to claim 6, wherein $X^p$ (np=1) is an N-terminal residue and $X^q$ (nq=1) is $X^{11}$.

**15.** A peptide having a cyclic structure or a pharmaceutically acceptable salt thereof according to claim 1, said peptide having an amino acid sequence shown by one of SEQ ID NOS: 4-7 and 16-32 in which one to several residues

which are the same or different and arbitrarily selected from the group consisting of organic acid residue, amino acid residues and a 12-aminododecanoic acid residue mentioned in the $X^i$ representations in claim 1 may be deleted, substituted or added.

**16.** A peptide having a cyclic structure or a pharmaceutically acceptable salt thereof according to claim 15, said peptide having an amino acid sequence shown by one of SEQ ID NOS: 4-7, 16, 19 and 25-32 in which one to several residues which are the same or different and arbitrarily selected from the group consisting of organic acid residues , amino acid residues and a 12-aminododecanoic acid residue mentioned in the $X^i$ representations in claim 1 may be deleted, substituted or added.

Fig. 1

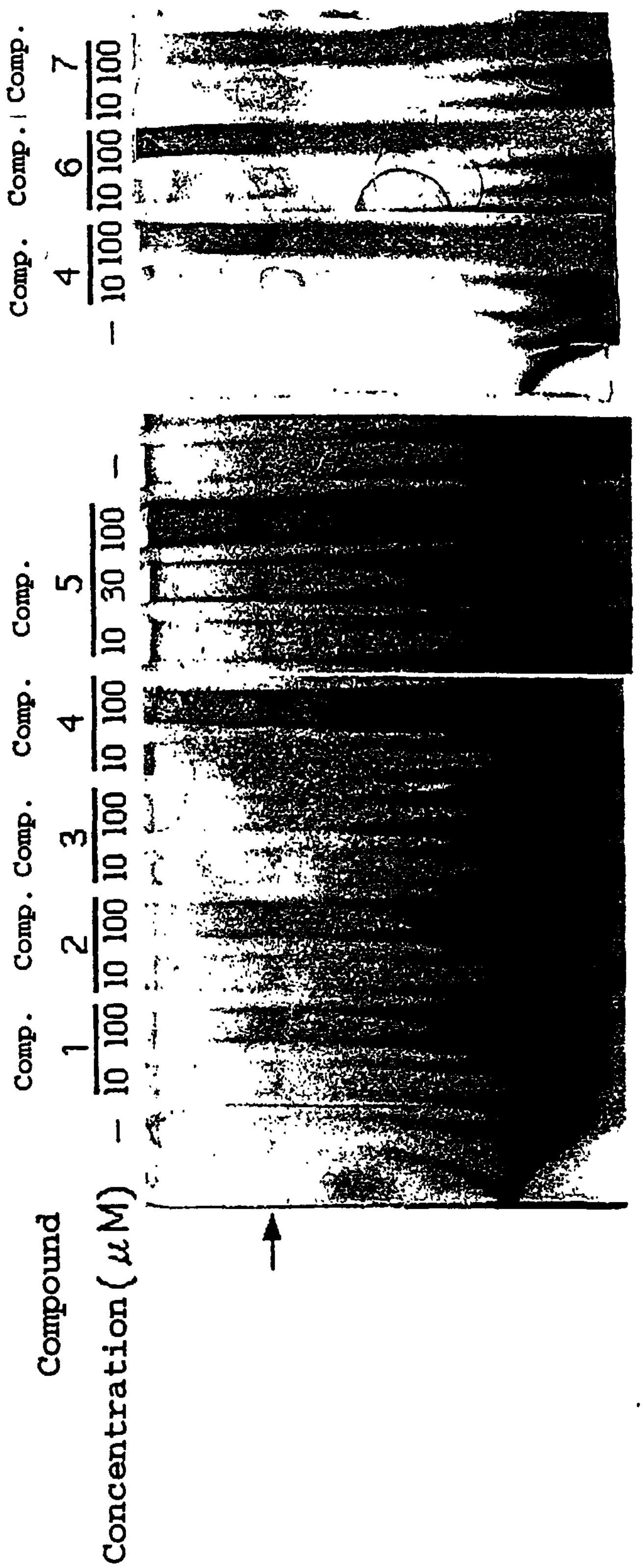
Compound
Concentration (μM)
–
Comp. 1
10 100
Comp. 2
10 100
Comp. 3
10 100
Comp. 4
10 100
Comp. 5
10 30 100
–
–
Comp. 4
10 100
Comp. 6
10 100
Comp. 7
10 100

Fig. 2

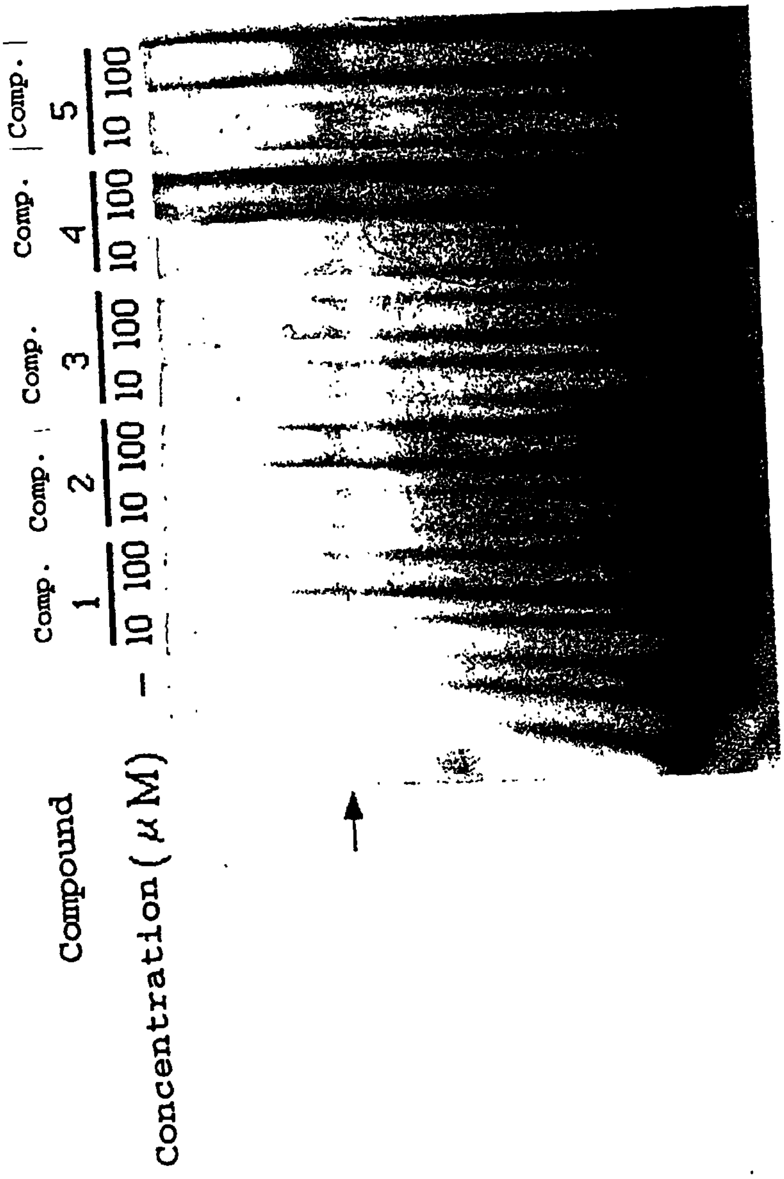
Compound
Concentration (μM)
Comp. 1
Comp. 2
Comp. 3
Comp. 4
Comp. 5
–
10 100
10 100
10 100
10 100
10 100

Fig. 3

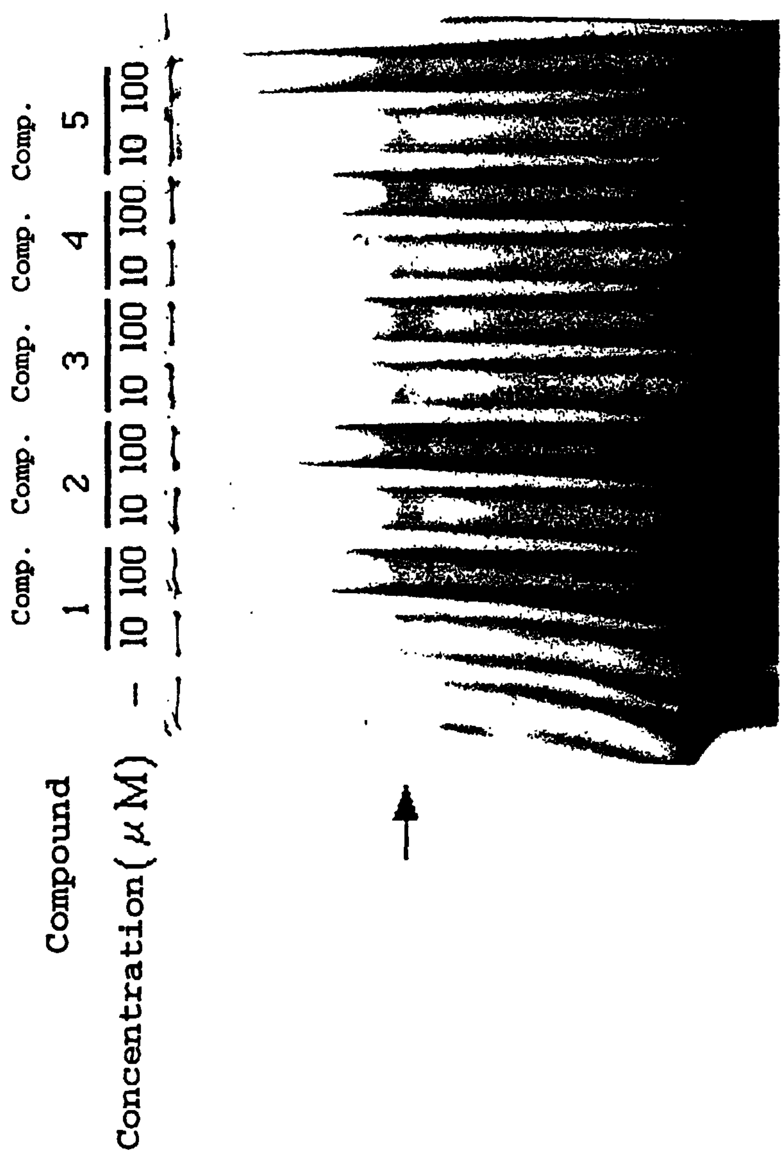
Compound
Concentration(μM)
Comp. 1
Comp. 2
Comp. 3
Comp. 4
Comp. 5
−
10 100
10 100
10 100
10 100
10 100

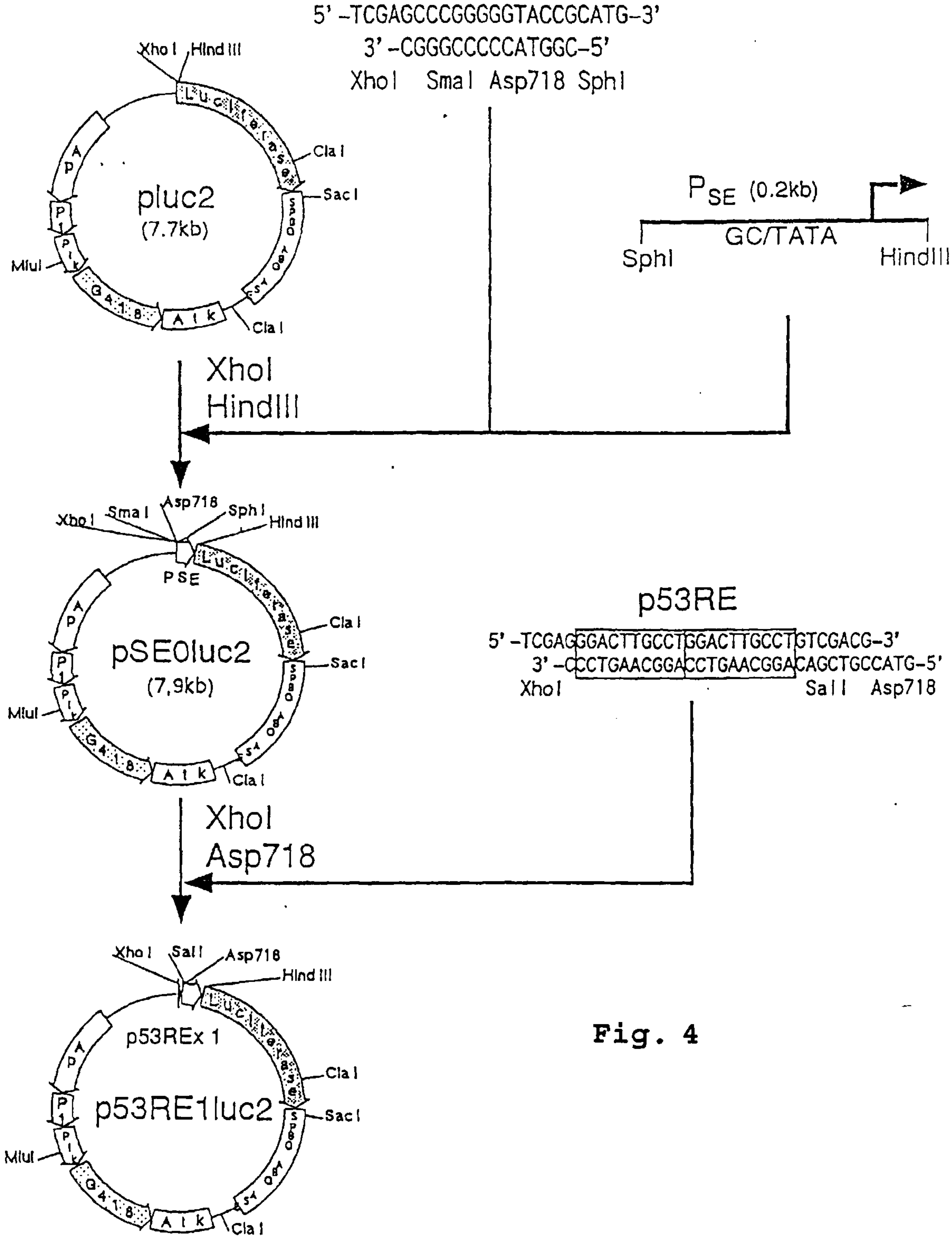
5'-TCGAGCCCGGGGGTACCGCATG-3'
3'-CGGGCCCCCATGGC-5'
XhoI SmaI Asp718 SphI
XhoI HindIII
Luciferase
ClaI
SacI
pluc2
(7.7kb)
MluI
G418
Atk
$P_{SE}$ (0.2kb)
GC/TATA
SphI
HindIII
XhoI
HindIII
XhoI SmaI Asp718 SphI HindIII
PSE
pSE0luc2
(7,9kb)
p53RE
5'-TCGAGGGACTTGCCTGGACTTGCCTGTCGACG-3'
3'-CCCTGAACGGACCTGAACGGACAGCTGCCATG-5'
XhoI SalI Asp718
XhoI
Asp718
XhoI SalI Asp718 HindIII
p53REx 1
p53RE1luc2

Fig. 4

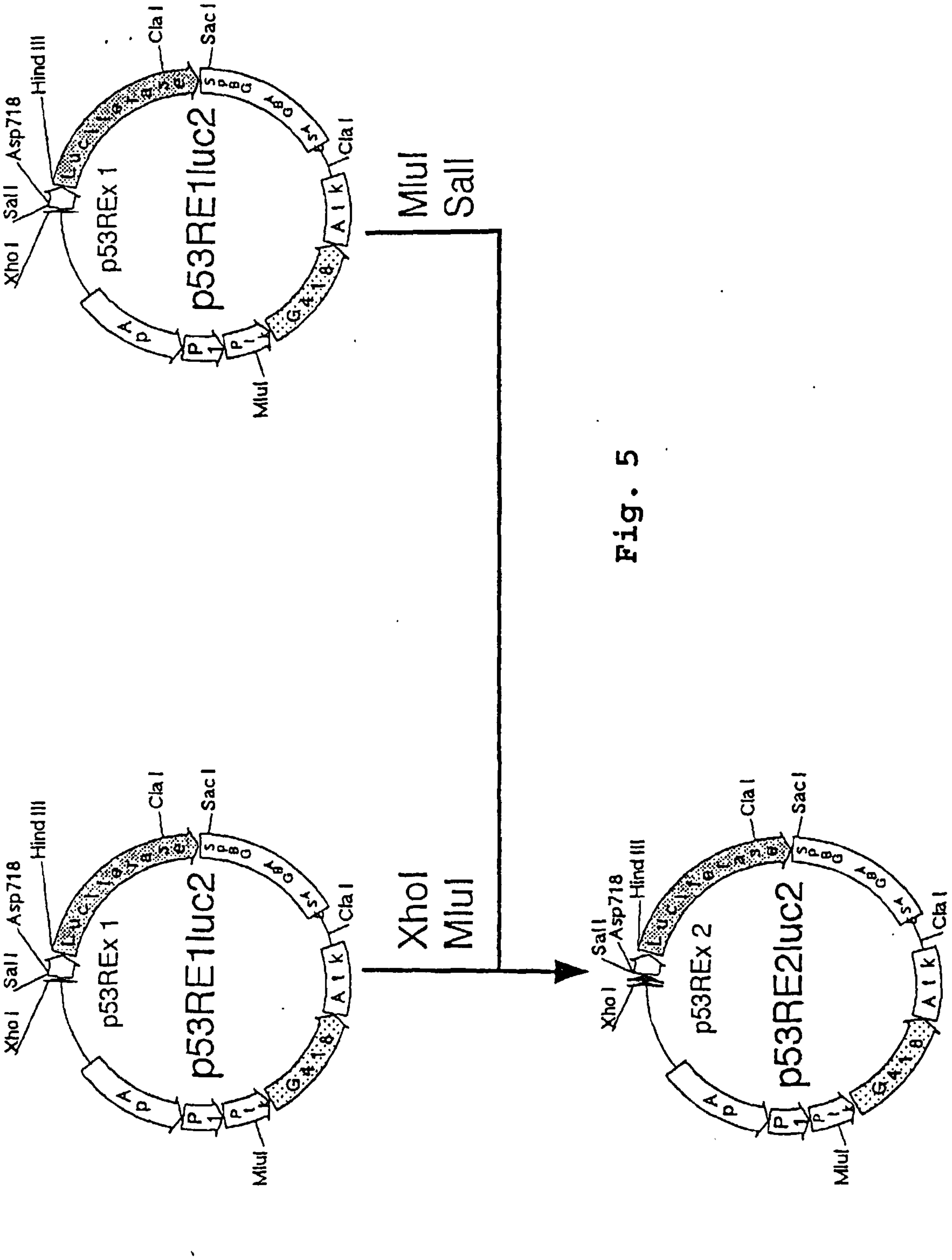
p53RE1luc2
p53REx 1
XhoI
SalI
Asp718
Hind III
Cla I
Sac I
Luciferase
Ap
Mlul
MluI
SalI
XhoI
MluI
p53RE2luc2
p53REx 2

Fig. 5

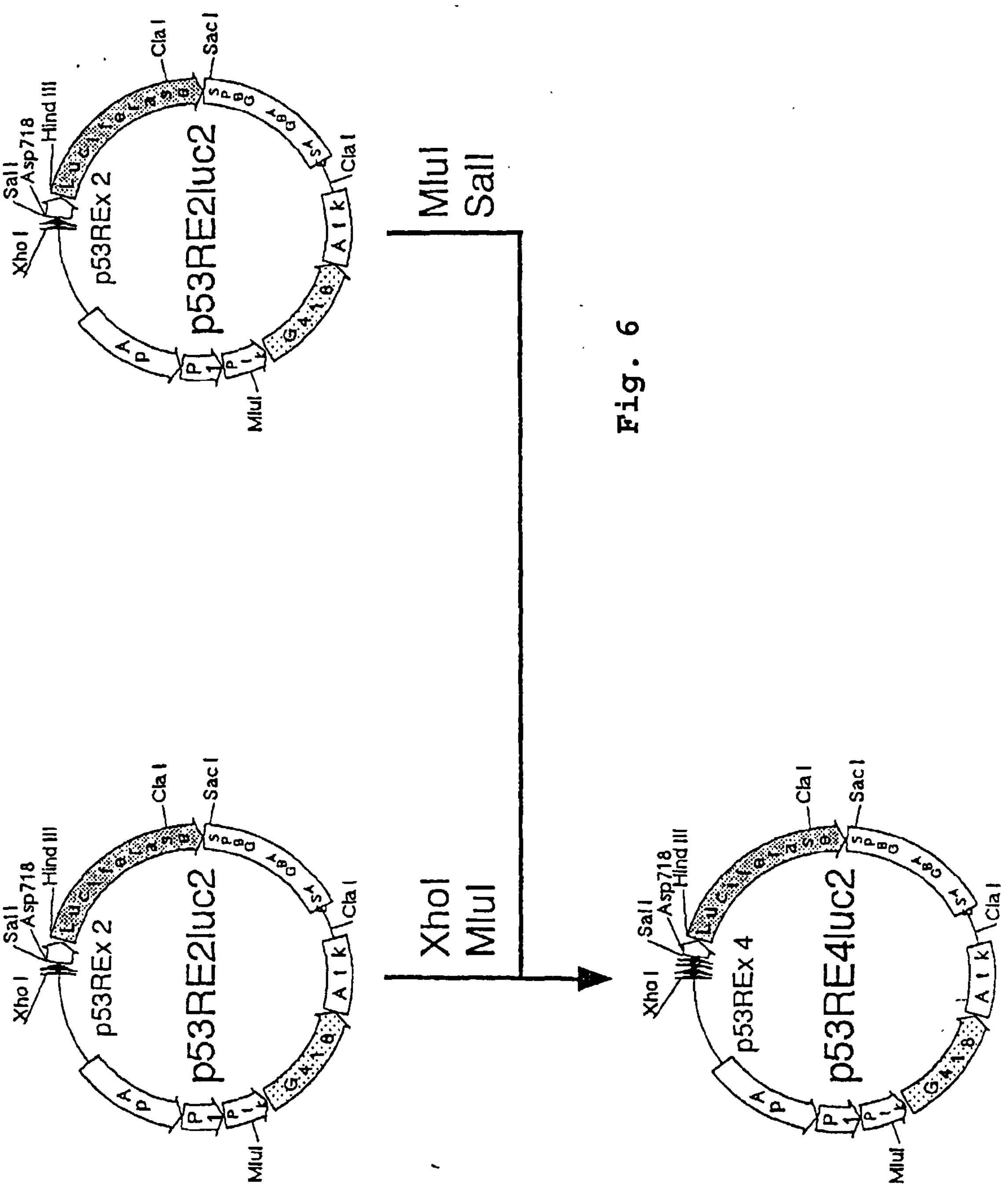
p53RE2luc2
p53REx 2
Xho I
Sal I
Asp718
Hind III
Cla I
Sac I
Mlul
Mlul
SalI
p53RE2luc2
p53REx 2
XhoI
MluI
p53RE4luc2
p53REx 4

Fig. 6

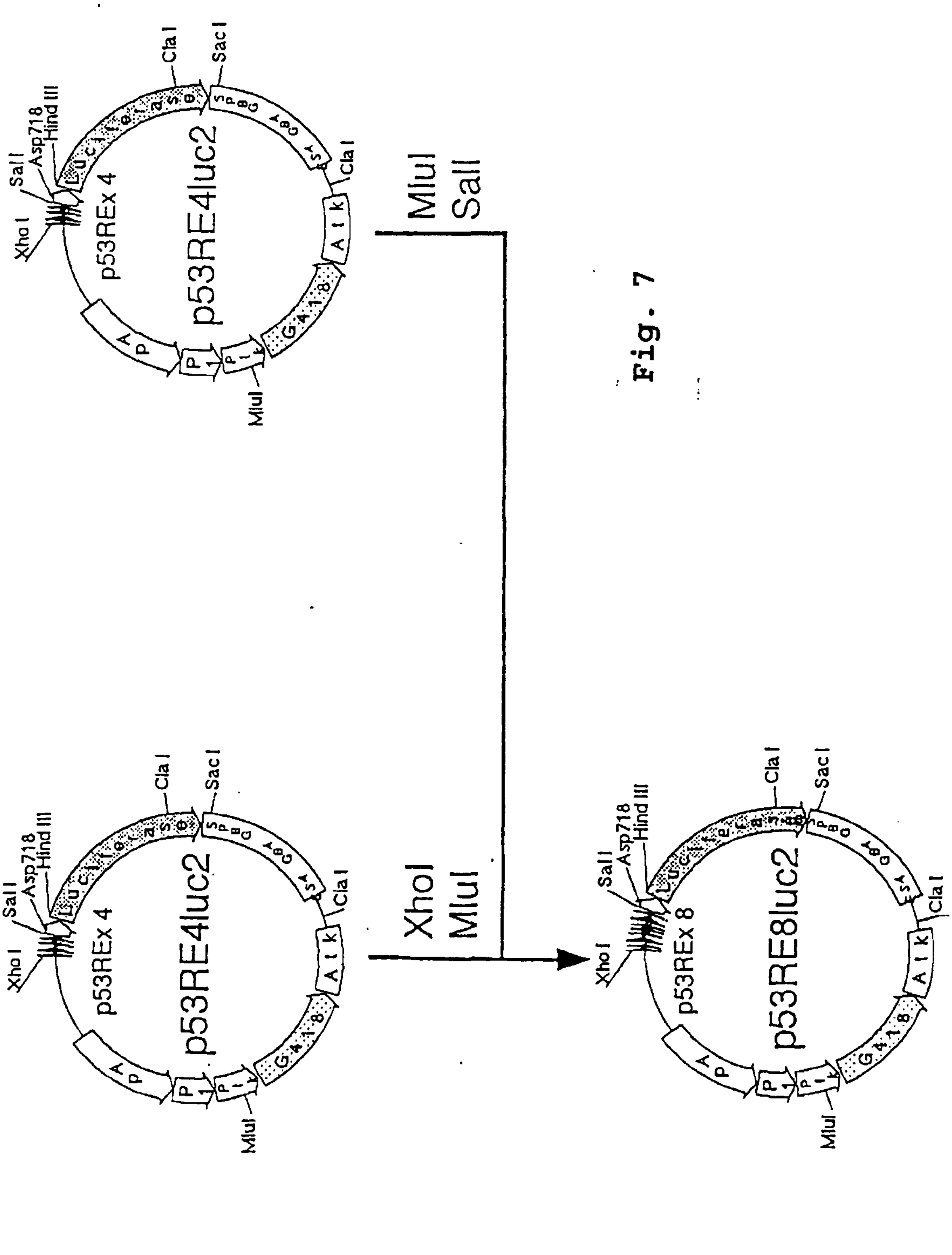
XhoI
SalI
Asp718
HindIII
ClaI
SacI
ClaI
MluI
p53REx 4
p53RE4luc2
MluI
SalI
XhoI
SalI
Asp718
HindIII
ClaI
SacI
ClaI
MluI
p53REx 4
p53RE4luc2
XhoI
MluI
XhoI
SalI
Asp718
HindIII
ClaI
SacI
ClaI
MluI
p53REx 8
p53RE8luc2

Fig. 7

# INTERNATIONAL SEARCH REPORT

International application No. PCT/JP98/02148

A. CLASSIFICATION OF SUBJECT MATTER
Int.Cl[6] C07K14/82 // C12N15/09, A61K38/17

According to International Patent Classification (IPC) or to both national classification and IPC

B. FIELDS SEARCHED

Minimum documentation searched (classification system followed by classification symbols)
Int.Cl[6] C07K14/82, A61K38/17

Documentation searched other than minimum documentation to the extent that such documents are included in the fields searched

Electronic data base consulted during the international search (name of data base and, where practicable, search terms used)
CA (STN), REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | WO, 96/25434, A1 (BAYER CORP.), August 22, 1996 (22. 08. 96) & EP, 809655, A1 | 1-16 |
| A | WO, 97/14794, A1 (UNIV. DUNDEE), April 24, 1997 (24. 04. 97) & AU, 9673174, A | 1-16 |
| A | SELIVANOVA, Galina et al., "Restration of the growth suppression function of mutant p53 by a synthetic peptide derived from the p53 C-terminal domain", Nat. Med. (N.Y.), 1997, 3(6), 632-638 | 1-16 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| July 23, 1998 (23. 07. 98) | August 4, 1998 (04. 08. 98) |
| Name and mailing address of the ISA/ Japanese Patent Office | Authorized officer |
| Facsimile No. | Telephone No. |

Form PCT/ISA/210 (second sheet) (July 1992)